# EUROPEAN PATENT SPECIFICATION

(11) **EP 2 011 860 B1**
(45) Date of publication and mention of the grant of the patent: **26.12.2012**
(21) Application number: 07741792.1
(22) Date of filing: 17.04.2007
(51) Int. Cl.: C12N 1/21, C12N 9/16, C12N 15/09, C12P 19/32, C12P 19/40, C12R 1/125

(54) **BACTERIUM CAPABLE OF PRODUCING PURINE SUBSTANCE, AND PROCESS FOR PRODUCTION OF PURINE SUBSTANCE**
BAKTERIUM MIT FÄHIGKEIT ZUR PRODUKTION EINER PURINSUBSTANZ UND VERFAHREN ZUR PRODUKTION DER PURINSUBSTANZ
BACTÉRIE CAPABLE DE PRODUIRE UNE SUBSTANCE PURINE ET PROCÉDÉ DE PRODUCTION D'UNE SUBSTANCE PURINE

(30) Priority: 24.04.2006 JP 2006119315
(43) Date of publication of application: 07.01.2009
(73) Proprietor: Ajinomoto Co., Inc., Tokyo 104-8315 (JP)
(72) Inventor: ASAHARA, Takayuki, Kawasaki-shi, Kanagawa 210-8681 (JP); MATSUNO, Kiyoshi, Kawasaki-shi, Kanagawa 210-8681 (JP); MORI, Yukiko, Kawasaki-shi, Kanagawa 210-8681 (JP)
(74) Representative: Strehl Schübel-Hopf & Partner
(86) International application number: PCT/JP2007/058356
(87) International publication number: WO 2007/125782

(56) References cited:
- EP-A- 1 004 663
- EP-A- 1 577 386
- EP-A- 2 011 861
- WO-A-03/083125
- WO-A-2006/033668
- JP-A- 2005 521 407
- GU ET AL: "Rv2131c gene product: An unconventional enzyme that is both inositol monophosphatase and fructose-1,6-bisphosphatase" BIOCHEMICAL AND BIOPHYSICAL RESEARCH COMMUNICATIONS, ACADEMIC PRESS INC. ORLANDO, FL, US, vol. 339, no. 3, 20 January 2006 (2006-01-20), pages 897-904, XP005198130 ISSN: 0006-291X
- FUJITA Y. ET AL.: 'Isolation and properties of a Bacillus subtilis mutant unable to produce fructose-bisphosphatase' J. BACTERIOL. vol. 145, no. 2, 1981, pages 760 - 767, XP009035077

## Description

### Technical Field

The present invention relates to a method for producing purine-derived substances such as purine nucleotides typically including 5'-inosinic acid and 5'-guanylic acid, and purine nucleosides such as inosine and guanosine, which are important substances as starting materials for synthesis of the purine nucleotides using a bacterium belonging to the genus *Bacillus* used for the method. Purine-derived substances are useful as seasonings, drugs, raw materials thereof, and so forth.

### Background Art

Methods for producing inosine and guanosine by fermentation using adenine auxotrophic strains of *Bacillus* bacteria and derivatives thereof which are further imparted with resistance to various drugs such as purine analogues (Patent documents 1 to 8), and such microorganisms of the genus *Brevibacterium* (Patent documents 9 and 10, Non-patent document 1), and so forth have been known.

Such mutant strains are typically obtained by treating a microorganism with ultraviolet irradiation or nitrosoguanidine (N-methyl-N'-nitro-N-nitrosoguanidine), and selecting a target mutant using a suitable selection medium.

Furthermore, strains which produce purine-derived substances have also been bred using genetic engineering techniques in *Bacillus* bacteria (Patent documents 11 to 20), *Brevibacterium* bacteria(Patent document 21), and *Escherichia* bacteria (Patent document 22). Specifically, for example, a method for efficiently producing a nucleic acid-derived compound such as hypoxanthine, uracil, guanine and adenine with a *Bacillus* bacterium in which a gene (*purR*) encoding the purine operon repressor is disrupted is disclosed (Patent document 23).

In *Bacillus subtilis,* the purine operon repressor as described above is known to regulate, besides the genes of purine operon, the *purA* gene which is involved in AMP biosynthesis (Non-patent document 2), the *glyA* gene which is involved in formyltetrahydrofolic acid biosynthesis (Non-patent document 3), the *pbuG* gene which encodes the transporter of hypoxanthine/guanine (Non-patent document 4), and so forth.

Furthermore, a microorganism which efficiently produces inosine derived by impartation of adenine auxotrophy by disruption of the succinyl-AMP synthase gene (*purA*) in addition to disruption of the *purR* gene, and suppression of decomposition of inosine into hypoxanthine by disruption of the purine nucleoside phosphorylase gene (*deoD*), and a method for producing inosine using the microorganism have been disclosed (Patent document 8).

Fructose bisphosphatase is one of the gluconeogenic enzymes, which catalyzes the reaction of generating fructose-6-phosphate from fructose-1,6-bisphosphate. There is only little knowledge about the relationship between this enzyme and the biosynthetic pathway of purine-derived substances, and it has not been attempted to breed a purine-derived substance-producing bacterium by reducing the activity of this enzyme.
Patent document 1: Japanese Patent Publication (KOKOKU) No. 38-23099
Patent document 2: Japanese Patent Publication No. 54-17033
Patent document 3: Japanese Patent Publication No. 55-2956
Patent document 4: Japanese Patent Publication No. 55-45199
Patent document 5: Japanese Patent Publication No. 57-14160
Patent document 6: Japanese Patent Publication No. 57-41915
Patent document 7: Japanese Patent Laid-open (KOKAI) No. 59-42895
Patent document 8: Japanese Patent Laid-open No. 2004-242610
Patent document 9: Japanese Patent Publication No. 51-5075
Patent document 10: Japanese Patent Publication No. 58-17592
Patent document 11: Japanese Patent Laid-open No. 58-158197
Patent document 12: Japanese Patent Laid-open No. 58-175493
Patent document 13: Japanese Patent Laid-open No. 59-28470
Patent document 14: Japanese Patent Laid-open No. 60-156388
Patent document 15: Japanese Patent Laid-open No. 1-27477
Patent document 16: Japanese Patent Laid-open No. 1-174385
Patent document 17: Japanese Patent Laid-open No. 3-58787
Patent document 18: Japanese Patent Laid-open No. 3-164185
Patent document 19: Japanese Patent Laid-open No. 5-84067
Patent document 20: Japanese Patent Laid-open No. 5-192164
Patent document 21: Japanese Patent Laid-open No. 63-248394
Patent document 22: International Patent Publication WO99/03988
Patent document 23: U.S. Patent No. 6,284,495
Non-patent document 1: Agric. Biol. Chem., 1978, 42, 399-405
Non-patent document 2: Proc. Natl. Acad. Sci. USA, 1995, 92, 7455-7459
Non-patent document 3: J. Bacteriol., 2001, 183, 6175-6183
Non-patent document 4: J. Bacteriol., 2003, 185, 5200-5209

### Disclosure of the Invention

### Object to be Achieved by the Invention

An object of the present invention is to construct a *Bacillus* bacterium suitable for fermentation production of purine-derived substances such as purine nucleosides and/or purine nucleotides, and to provide a method for producing a purine-derived substance using such a bacterium.

### Means for Achieving the Object

The inventors of the present invention conducted various researches in order to achieve the aforementioned object. As a result, they found that if the enzymatic activity of fructose bisphosphatase of the glyconeogenesis pathway was decreased in a *Bacillus* bacterium, an ability of the bacterium to produce a purine nucleoside or a purine nucleotide is improved, and accomplished the present invention.

The present specification thus discloses the following.
(1) A bacterium belonging to the genus *Bacillus* having an ability to produce purine-derived substance, wherein the bacterium has been modified so that enzymatic activity of fructose bisphosphatase is decreased.
(2) The bacterium belonging to the genus *Bacillus* as described above, wherein the purine-derived substance is a purine nucleoside selected from the group consisting of inosine, xanthosine, guanosine and adenosine.
(3) The bacterium belonging to the genus *Bacillus* as described above, wherein the purine-derived substance is a purine nucleotide selected from the group consisting of inosinic acid, xanthylic acid, guanylic acid and adenylic acid.
(4) The bacterium belonging to the genus *Bacillus* as described above, wherein the fructose bisphosphatase activity is decreased by disruption of a gene encoding fructose bisphosphatase.
(5) The bacterium belonging to the genus *Bacillus* as described above, wherein the gene encoding fructose bisphosphatase is a gene encoding a protein of the following (A) or (B):
   (A) a protein comprising the amino acid sequence of SEQ ID NO: 1,
   (B) a protein comprising the amino acid sequence of SEQ ID NO: 1 which includes substitutions, deletions, insertions, additions or inversions of one or several amino acid residues and has fructose bisphosphatase activity.
(6) The bacterium belonging to the genus *Bacillus* as described above, which has been further modified so that phosphoribosyl pyrophosphate synthetase activity is increased.
(7) The bacterium belonging to the genusBacillus as described above, which has been further modified so that expression amount of the purine operon is increased.
(8) The bacterium belonging to the genus *Bacillus* as described above, wherein expression amount of the purine operon is increased by disruption of the *purR* gene, which is a gene encoding a repressor of the purine operon.
(9) The bacterium belonging to the genus *Bacillus* as described above, which has been further modified so that purine nucleoside phosphorylase activity is decreased.
(10) The bacterium belonging to the genus *Bacillus* as described above, which has been further modified so that IMP dehydrogenase activity is decreased.
(11) The bacterium belonging to the genus *Bacillus* as described above, which is *Bacillus subtilis.*
(12) A method for producing a purine-derived substance, which comprises culturing the bacterium belonging to the genus *Bacillus* as described above in a medium to cause accumulation of a purine-derived substance in cells of the bacterium or the medium, and collecting the purine-derived substance from the cells or medium.
(13) The method as described above, wherein the purine-derived substance is a purine nucleoside or a purine nucleotide.
(14) The method as described above, wherein the purine-derived substance is a purine nucleoside selected from the group consisting of inosine, xanthosine, guanosine and adenosine.
(15) The method as described above, wherein the purine-derived substance is a purine nucleotide selected from the group consisting of inosinic acid, xanthylic acid, guanylic acid and adenylic acid.
(16) A method for producing a purine nucleotide, which comprises producing a purine nucleoside by the method as described above, reacting the purine nucleoside with a phosphate donor selected from the group consisting of polyphosphoric acid, phenyl phosphate and carbamyl phosphate, and a microorganism having an ability to produce a nucleoside-5'-phosphoric acid ester or acid phosphatase to produce a purine nucleotide, and collecting the purine nucleotide.

### Best Mode for Carrying out the Invention

### <1> Bacillus bacterium

### (I) Impartating the ability to produce a purine-derived substance

In the present invention, the phrase "activity is decreased" encompasses a state that the activity is lower than the activity in a ummodified strain such as a wild-type *Bacillus* bacterium, and a state that the activity has substantially eliminated.

The *Bacillus* bacterium described herein is a *Bacillus* bacterium which has the ability to produce a purine-derived substance and has been modified so that enzymatic activity of fructose bisphosphatase is decreased.

The term "purine-derived substance" means a substance having a purine skelton, and examples thereof include purine nucleosides, purine nucleotides, and so forth. The purine nucleosides include inosine, xanthosine, guanosine, adenosine, and so forth, and the purine nucleotides include 5'-phosphoric acid esters of purine nucleosides, for example, inosinic acid (inosine-5'-phosphate, henceforth also referred to as "IMP"), xanthylic acid (xanthosine-5'-phosphate, henceforth also referred to as "XMP"), guanylic acid (guanosine-5'-monophosphate, henceforth also referred to as "GMP"), adenylic acid (adenosine-5'-monophosphate, henceforth also referred to as "AMP"), and so forth.

The phrase "ability to produce a purine-derived substance " means an ability of the *Bacillus* bacterium described herein to produce, secrete or cause accumulation of a purine-derived substance in the bacterial cells or a medium when the bacterium is cultured in the medium to such an extent that the purine-derived substance can be collected from the cells or medium. The *Bacillus* bacterium descrideb herein may have an ability to produce two or more kinds of the aforementioned purine-derived substances.

The *Bacillus* bacterium having the ability to produce a purine-derived substance may be a bacterium inherently having the ability to produce a purine-derived substance, or a bacterium obtainable by modifying such *Bacillus* bacteria as described below using a mutagenesis or recombinant DNA technique so that they have the ability to produce a purine-derived substance. Moreover, it may be a *Bacillus* bacterium to which the ability to produce a purine-derived substance is imparted or of which the ability to produce a purine-derived substance is enhanced by modifying the bacterium so that enzymatic activity of fructose bisphosphatase isdecreased, in such a manner as described later.

In the present invention, the prase "enzymatic activity is decreased" encompasses a state that enzymatic activity of fructose bisphosphatase described above, or an enzyme which decomposes a purine-derived substance described later such as inosine monophosphate (IMP) dehydrogenase, or the like is lower than that in an unmodified strain, for example, a wild-type strain of the *Bacillus* bacterium, and a state that the activity has substantially eliminated. The same shall apply to the activity of the purine operon repressor described later.

Examples of the *Bacillus* bacterium used for the *Bacillus* bacterium of the present method include *Bacillus subtilis, Bacillus amyloliquefaciens, Bacillus pumilus*.

Examples of *Bacillus subtilis* include *Bacillus subtilis* 168 Marburg (ATCC 6051), *Bacillus subtilis* PY79 (Plasmid, 1984, 12, 1-9) and so forth, and examples of *Bacillus amyloliquefaciens* include *Bacillus amyloliquefaciens* T (ATCC 23842), *Bacillus amyloliquefaciens* N (ATCC 23845), and so forth. Examples of *Bacillus pumilus* include *Bacillus pumilus* Gottheil No. 3218 (ATCC No. 21005, U.S. Patent No. 3,616,206), and so forth. These strains can be obtained from the American Type Culture Collection (Address: P.O. Box 1549, Manassas, VA 20108, United States of America).

A *Bacillus* bacterium having the ability to produce a purine-derived substance can be obtained by imparting, for example, purine nucleoside auxotrophy or resistance to a drug such as purine analogue to such *Bacillus* bacteria as described above (Japanese Patent Publication Nos. 38-23099, 54-17033, 55-45199, 57-14160, 57-41915 and 59-42895). A *Bacillus* bacterium having auxotrophy or drug resistance can be obtained by treating the bacterium with mutagen which is used for usual mutagenesis such as N-methyl-N'-nitro-N-nitrosoguanidine (NTG) or EMS (ethyl methanesulfonate).

Examples of *Bacillus* bacteria which produce a purine nucleoside include the followings.

As a specific example of inosine-producing strain belonging to the genus *Bacillus,* the *Bacillus subtilis* KMBS16 strain can be used. This strain is derived from the known *Bacillus subtilis* trpC2 strain (168 Marburg), wherein a *purR* gene encoding the purine operon repressor (purR::spc), a *purA* gene encoding succinyl-AMP synthase (purA::erm), and a *deoD* gene encoding purine nucleoside phosphorylase (deoD::kan) are disrupted (Japanese Patent Laid-open No. 2004-242610, US2004166575A1). *Bacillus subtilis* AJ3772 strain (FERM P-2555, Japanese Patent Laid-open No. 62-014794) and so forth may also be used.

Examples of *Bacillus* bacteria which has an ability to prouduce guanosine include a *Bacillus* bacterium which has increased IMP dehydrogenase activity (Japanese Patent Laid-open No. 3-58787), a *Bacillus* bacterium which is obtained by introducing a vector comprising a gene conferring resistance to purine analogue or decoyinine into an adenine auxotrophic mutant (Japanese Patent Publication No. 4-28357), and so forth.

Examples of *Bacillus* bacteria which produce a purine nucleotide include the followings.

As an inosinic acid-producing *Bacillus* bacteria, inosine-producing strains of *Bacillus subtilis* which have attenuated phosphatase activity have been reported (Uchida, K. et al., Agr. Biol. Chem., 1961, 25, 804-805; Fujimoto, M., Uchida, K., Agr. Biol. Chem., 1965, 29, 249-259). Examples of guanylic acid-producing *Bacillus* bacteria include mutants of *Bacillus* bacteria which have adenine auxotrophy, resistance to decoyinine or methionine sulfoxide, and an ability to produce 5'-guanylic acid (guanosine-5'-monophosphate, henceforth referred to as "GMP") (Japanese Patent Publication No. 56-12438).

Furthermore, a xanthylic acid-producing bacterium can be constructed by the method used for breeding of coryneform bacteria tycially including *Corynebacterium ammoniagenes*. For example, by obtaining a PRPP amidotransferase-enhanced strain (Japanese Patent Laid-open No. 8-168383), an aliphatic amino acid-resistant strain (Japanese Patent Laid-open No. 4-262790), or a dehydroproline-resistant strain (South Korean Patent Unexamined Publication No. 2003-56490), a xanthylic acid-producing bacterium can be constructed.

Moreover, example of a method for breeding *Bacillus* bacteria which have the ability to produce a purine-derived substance also includes enhancing the activitis of enzymes involved in purine biosynthesis which are common to the biosynthesis of purine nucleosides and purine nucleotides, i.e., purine biosynthesis enzymes, in bacterial cells. The activity of the enzyme in the cells is preferably increased to a level greater than that of an unmodified strain of *Bacillus* bacterium, for example, a wild-type *Bacillus* bacterium. The phrase "activity is increased" encompasses, for example, when number of enzyme molecules per cell is increased, and when the specific activity per enzyme molecule is increased, and so forth. For example, the activity can be increased by increasing expression amount of the gene of the enzyme.

Examples of an enzyme involved in the purine biosynthesis include, for example, phosphoribosyl pyrophosphate amidotransferase, phosphoribosyl pyrophosphate synthetase (PRPP synthetase [EC: 2.7.6.1]), and so forth.

Some of the catabolites produced by metabolism of sugar sources such as glucose that flow into the pentose phosphate pathway are converted into ribose-5-phosphate via ribulose-5-phosphate. From the biosynthesized ribose-5-phosphate, PRPP is produced, which is an indispensable precursor for purine nucleoside, histidine and tryptophan biosyntheses. Specifically, ribose-5-phosphate is converted into PRPP by phosphoribosyl pyrophosphate synthetase. Therefore, an ability to produce purine-derived substance can be imparted to a *Bacillus* bacterium by modifying so that the activity of PRPP synthetase thereof is increased.

The phrase "activity of phosphoribosyl pyrophosphate synthetase is increased" means that the activity of phosphoribosyl pyrophosphate synthetase increases as compared to that of an unmodified strain such as a wild-type strain or a parent strain. The activity of the phosphoribosyl pyrophosphate synthetase can be measured by, for example, the method of Switzer et al. (Methods Enzymol., 1978, 51, 3-11) or Roth et al. (Methods Enzymol., 1978, 51, 12-17). A *Bacillus* bacterium in which the activity of phosphoribosyl pyrophosphate synthetase is increased can be produced by, for example, increasing expression of a gene encoding the phosphoribosyl pyrophosphate synthetase in a *Bacillus* bacterium according to a method of using a plasmid containing the gene or integrating the gene into a chromosome, which can be performed in the same manner as that of the method described in Japanese Patent Laid-open No. 2004-242610. Although the *prs* gene of SEQ ID NO: 3 derived from a *Bacillus* bacterium (Genbank Accession No. X16518) can be mentioned as the gene encoding phosphoribosyl pyrophosphate synthetase usable for the present invention, any of genes derived from other bacteria, animals or plants encoding a protein having the phosphoribosyl pyrophosphate synthetase activity may be used.

Furthermore, when PRPP which is an indispensable precursor for purine nucleoside, histidine and tryptophan biosyntheses is once produced, some of it is converted into purine nucleotides and purine nucleosides by the enzymes involved in the purine biosynthesis. Examples of genes encoding such enzymes include the genes of the purine operon from *Bacillus subtilis,* specifically, genes of the purEKB-purC(orf) QLF-purMNH(J)-purD operon (Ebbole D.J. and Zalkin H., J. Biol. Chem., 1987, 262, 17, 8274-87) (at present, also called purEKBCSQLFMNHD, *Bacillus subtilis* and Its Closest Relatives, Editor in Chief: A.L. Sonenshein, ASM Press, Washington D.C., 2002, Genbank Accession No. NC_000964), and the genes of the pur regulon from *Escherichia coli* (Escherichia and Salmonella, Second Edition, Editor in Chief: F.C. Neidhardt, ASM Press, Washington D.C., 1996).

Accodingly, enhancing expression of these genes imparts or enhances the ability to produce a purine-derived substance. In addition, genes of the purine operon which can be used for the present invention are not limited to these, and genes derived from other microorganisms, animals and plants may also be used.

Examples of the method for increasing expression amount of the purine operon include increasing expression of genes of the purine operon in a *Bacillus* bacterium by a method of using a plasmid containing the genes or integrating the genes into a chromosome or the like.

The second method for increasing expression amount of the purine operon includes replacing a promoter of the purine operon with a stronger one, and replacing the -35 or -10 region of the native promoter with a consensus sequence.

For example, in *Bacillus subtilis* (*B*. *subtilis* 168 Marburg strain, ATCC 6051), the -35 sequence of the purine operon is a consensus sequence (TTGACA), but the -10 sequence is TAAGAT, which differs from the consensus sequence TATAAT (Ebbole, D.J. and H. Zalikn, J. Biol. Chem., 1987, 262, 8274-8287). Therefore, by changing the -10 sequence (TAAGAT) to the similar consensus sequence TATAAT, TATGAT or TAAAAT, the transcriptional activity of the purine operon can be increased. A promoter sequence can be replaced by the same method as that of the gene substitution, which is described below.

The third method for increasing amount of expression of the purine operon includes reducing expression amount of the purine operon repressor (U.S. Patent No. 6,284,495). The phrase "expression of a purine operon repressor" includes both transcription of a purine operon gene and translation of a transcription product. Furthermore, "expression amount is decreased" include when the expression amount is lower than that in an unmodified strain such as a wild-type *Bacillus* bacterium, and when the expression has substantially eliminated.

Expression amount of the purine operon repressor (purine repressor) can be decreased by, for example, treating a *Bacillus* bacterium with ultraviolet ray irradiation or mutagen used in a usual mutagenesis treatment such as NTG or EMS and selecting a mutant showing decreased expression amount of the purine repressor can be employed.

Furthermore, a *Bacillus* bacterium exhibiting a decreased expression amount of the purine repressor can also be obtained by, for example, besides a mutagenesis treatment, replacing a gene encoding the purine repressor on a chromosome (*purR*, GenBank Accession NC_000964, coding region corresponds to the nucleotide numbers 54439 to 55293, SEQ ID NO: 5) with a corresponding gene that does not normally function (hereafter, also referred to as "disrupted-type gene") by homologous recombination utilizing a gene recombination technique (Experiments in Molecular Genetics, Cold Spring Harbor Laboratory Press (1972); Matsuyama, S. and Mizushima, S., J. Bacteriol., 1985, 162, 1196-1202).

For example, a disrupted-type gene for a normal gene can be replaced with a disrupted-type gene on the host chromosome in the mannaer as described below. Herein after, the disruption of the *purR* gene is explained. Other genes such as the *purA*, *deoD, guaB* and *fbp* genes can be similarly disrupted.

When a plasmid or the like that has a sequence homologous with a sequence on the chromosome and which is not capable of replicating in cells of *Bacillus* bacteria is introduced into the cell, resulting in recombination at the site of the sequence having homology at a certain frequency. The entire plasmid is then recombined into the chromosome. Thereafter, if recombination is further caused at the site of the sequence having homology on the chromosome, the plasmid is removed from the chromosome. At this time, depending on the site where the recombination occurs, the disrupted-type gene may be kept on the chromosome, and the original normal gene may be removed from the chromosome with the plasmid. By selecting such a strain, it is possible to obtain a strain in which the normal *purR* gene on the chromosome has been replaced with the disrupted-type *purR* gene.

Such gene disruption techniques based on homologous recombination has already been established and include a method of utilizing a linear DNA, a method of utilizing a temperature sensitive plasmid and so forth. Furthermore, the *purR* gene can also be disrupted by using a plasmid which contains the *purR* gene into which a marker gene such as a drug resistance gene has been inserted and which is not able to replicate in the target bacterial cell. That is, in a transformant that has been transformed with such a plasmid as described above, the marker gene is incorporated into the chromosomal DNA and imparts drug resitance. Since the marker gene is incorporated into the chromosome at a high rate by homologous recombination of the *purR* gene sequences that sandwiches the marker gene on the plasmid with the *purR* gene on the chromosome, a *purR* gene-disrupted strain can be efficiently selected.

The disrupted-type *purR* gene used for the gene disruption can be obtained by, specifically, deleting a certain region of the *purR* gene by digestion with a restriction enzyme and re-ligation, inserting another DNA fragment (marker gene etc.) into the *purR* gene, or causing substitution, deletion, insertion, addition or inversion of one or more nucleotides in the nucleotide sequence of the coding region, promoter region or the like of the *purR* gene by a site-specific mutagenesis (Kramer, W. and Frits, H.J., Methods in Enzymology, 1987, 154, 350-367) or by recombinant PCR (PCR Technology, Stockton Press (1989)) or by treantment with a chemical agent such as sodium hyposulfite or hydroxylamine (Shortle, D. and Nathans, D., Proc. Natl. Acad. Sci. U.S.A., 1978, 75, 2170-2174), followed by selection of a strain in which the activity of the repressor encoded by the *purR* gene is decreased or transcription of the gene is decreased. Among these methods, deleting a certain region of the *purR* gene by digestion with a restriction enzyme and re-ligation or inserting another DNA fragment into the *purR* gene is preferable in view of reliability and stability. The certain region of the *purR* gene to be deleted may be a 5' end sequence, internal sequence or 3' end sequence of the *purR* gene. However, if the region accounts for 90% or more, more preferably 95% or more, particularly 97% or more, of the full length of the *purR* gene, certainty of reduction of the repressor activity is increased. Furthermore, when a frame shift mutation is caused by deletion or insertion of nucleotides in the coding region of the *purR* gene, it is preferable to cause deletion or insertion of nucleotides at multiple sites and cause deletion or insertion of nucleotides on the 3' end side, in view of surely reducing the repressor activity.

Reduction of the purine repressor activity can also be attained by, besides the aforementioned gene disruption, introduction of a mutation that reduces the intracellular purine repressor activity into the *purR* gene on the chromosome based on a conventional mutagenesis method. For example, it can be attained by introducing an amino acid substitution (missense mutation), a stop codon (nonsense mutation), or a frame shift mutation that adds or deletes one or two nucleotides, or by deleting the gene partially or entirely. Furthermore, the activity of the repressor can also be decreased by inserting a transposon into the *purR* gene on the chromosome.

Reduction of the activity of the purine repressor can also be attained by replacing an expression control sequence of the *purR* gene such as promoter on the chromosomal DNA with a weaker one. The strength of a promoter is defined by the frequency of initiation acts of RNA synthesis. Examples of method for evaluating the strength of promoters and strong promoters are described in the paper of Goldstein et al. (Prokaryotic promoters in biotechnology, Biotechnol. Annu. Rev., 1995, 1, 105-128), and so forth. Furthermore, it is also possible to introduce nucleotide substitution for several nucleotides in a promoter region of a target gene and thereby modify the promoter to be weakened as disclosed in International Patent Publication WO00/18935. Furthermore, it is known that substitution of several nucleotides in the spacer between the ribosome binding site (RBS) and the start codon, in particular, a sequence immediately upstream from the start codon, greatly affects translation efficiency of mRNA. This modification of RBS may be combined with decreasing transcription of a target gene.

Furthermore, a recombinant DNA into which a mutation that unstabilizes messenger RNA transcribed from the *purR* gene is introduced may be prepared and transformed into a host *Bacillus* bacterium.

Activities of enzymes encoded by the *purA, deoD, guaB,* and *fbp* genes described later can also be decreased in the same manner as described above.

The *purR* gene can be obtained from a chromosomal DNA of a microorganism having the purine operon by PCR using oligonucleotides prepared based on the known nucleotide sequence of the *purR* gene as primers. The *purR* gene can also be obtained from a chromosomal DNA library of a microorganism having the purine operon by hybridization using an oligonucleotide prepared on the basis of the known nucleotide sequence of the *purR* gene as a probe. The nucleotide sequence of the *purR* gene of the *Bacillus subtilis* 168 Marburg strain has been reported (GenBank accession No. D26185 (the coding region corresponds to the nucleotide numbers 118041 to 118898), or NC_000964 (the coding region corresponds to the nucleotide numbers 54439 to 55296)). The nucleotide sequence of the *purR* gene and the amino acid sequence encoded by the gene are shown in SEQ ID NOS: 5 and 6 of Sequence Listing (Japanese Patent Laid-open No. 2004-242610).

Primers used for PCR for obtaining the *purR* gene may be any of those enabling amplification of a part or full length of the *purR* gene, and specific examples include oligonucleotides having the nucleotide sequences shown in SEQ ID NO: 15 (GAAGTTGATGATCAAAA) and SEQ ID NO: 16 (ACATATTGTTGACGATAAT).

Examples of the marker gene described above include drug resistance genes such as a spectinomycin resistance gene and kanamycin resistance gene. The spectinomycin resistance gene of *Enterococcus faecalis* can be obtained by preparing the plasmid pDG1726 from the *Escherichia coli* ECE101 strain commercially available from the *Bacillus* Genetic Stock Center (BGSC) and removing the resistance gene as a cassette from the plasmid. The erythromycin resistance gene of *Staphylococcus aureus* can be obtained by preparing the plasmid pDG646 from the *Escherichia coli* ECE91 strain commercially available from the *Bacillus* Genetic Stock Center (BGSC) and removing the resistance gene as a cassette from the plasmid. The kanamycin resistance gene derived from *Streptococcus faecalis* can be obtained by preparing the plasmid pDG783 from the *Escherichia coli* ECE94 strain commercially available from the *Bacillus* Genetic Stock Center (BGSC) and removing the resistance gene as a cassette from the plasmid. Furthermore, the chloramphenicol resistance gene of *Staphylococcus aureus* can be obtained by preparing the plasmid pC194 from the *Bacillus subtilis* 1E17 strain commercially available from the *Bacillus* Genetic Stock Center (BGSC) and performing PCR using that plasmid as a template to amplify the plasmid.

When a drug resistance gene is used as the marker gene, a *purR* gene-disrupted strain can be obtained by inserting the drug resistance gene into the *purR* gene in a plasmid at an appropriate site, transforming a microorganism with the obtained plasmid and selecting a transformant that has become drug resistant. Disruption of the *purR* gene on the chromosome can be confirmed by analyzing the *purR* gene or the marker gene on the chromosome using Southern blotting or PCR. Incorporation of the aforementioned spectinomycin resistance gene, erythromycin resistance gene or kanamycin resistance gene into a chromosomal DNA can be confirmed by PCR using primers which can amplify these genes.

It is also known that expression of the purine operon is regulated by the terminator-antiterminator sequence locating downstream of the promoter (henceforth referred to as attenuator sequence) (Ebbole, D.J. and Zalkin, H., J. Biol. Chem., 1987, 262, 8274-8287; Ebbole D.J. and Zalkin H., J. Biol. Chem., 1988, 263, 10894-10902; Ebbole, D.J. and Zalkin, H., J. Bacteriol., 1989, 171, 2136-2141). Therefore, expression amount of the purine operon can be increased by deleting the attenuator sequence. Deletion of the attenuator sequence can be attained by the same method as that for disruption of *purR*.

In order to further increase transcription of the purine operon, the methods described above may be combined. For example, the *purR* gene may be disrupted, and further, the purine operon from which the attenuator sequence is deleted may be amplified using a plasmid or multiple copies of such a purine operon may be introduced into a chromosome.

The activities of an enzyme involoved in purine biosynthesis can also be enhanced by desensitizing the enzyme involved in purine biosynthesis to regulation thereof, for example, according to the aforementioned method for desensitizing an enzyme to feedback inhibition (WO99/03988).

Furthermore, the ability to produce purine-derived substance can also be enhanced by attenuating uptake of purine-derived substances by the cells. For example, the uptake of purine nucleosides by the cells can be attenuated by blocking a reaction involved in the uptake of purine nucleosides by the cells. An example of the reaction involved in the uptake of the purine nucleosides by the cells includes reactions catalyzed by nucleoside permeases.

Furthermore, when a purine nucleoside is produced, activity of an enzyme which decomposes the purine nucleoside may be decreased in order to enhance the ability to produce a purine nucleoside. An examples of such an enzyme includes purine nucleoside phosphorylase.

Purine nucleotides biosynthesized from PRPP by enzymes involved in purine biosynthesis are dephosphorylated and thereby converted into a purine nucleoside. To efficiently cause accumulation of a purine nucleoside, it is preferable to reduce an activity of purine nucleoside phosphorylases, which further degrade purine nucleosides into hypoxanthine or the like. That is, it is preferable to attenuate or eliminate an activity of a purine nucleoside phosphorylase that employs purine nucleosides, such as inosine, as a substrate.

Specifically, the purine nucleoside phosphorylase activity can be decreased by disrupting the *deoD* and *pupG* genes encoding purine nucleoside phosphorylase in *Bacillus* bacteria. The *Bacillus* bacterium used in the present method may be modified by disrupting one or both of the *deoD* and *pupG* genes. As the *deoD* and the *pupG* genes, for example, those genes derived from *Bacillus* bacteria (deoD: Genbank Accession No. NC_000964 (SEQ ID NO: 7), *pupG*: Genbank Accession No. NC_000964 (SEQ ID NO: 9)) can be used, and a gene-disrupted strain can be obtained in the same manner as that used for the aforementioned disruption of the *purR* gene.

The ability to produce a purine-derived substance may also enhanced by decreasing the activity of succinyl-AMP synthase. An example of the gene encoding succinyl-AMP synthase includes the *purA* gene. A example of the *purA* gene includes, for example, those having the nucleotide sequence registered as GenBank Accession No. NC_000964 (coding region corresponds to the nucleotide numbers 4153460 to 4155749 of the complementary strand, SEQ ID NO: 11).

The ability to produce a purine-derived substance may also be enhanced by decreasing an activity of inosine monophosphate (IMP) dehydrogenase. An example of the gene encoding IMP dehydrogenase includes the *guaB* gene. An example of the *guaB* gene includes, for example, those having the nucleotide sequence registered as GenBank Accession No. NC_000964 (coding region corresponds to the nucleotide numbers 15913 to 17376, SEQ ID NO: 13).

Moreover, as a method for enhancing an ability to produce purine-derived substance, amplification of a gene encoding a protein having an activity of secreting a purine-derived substance may be contemplated. An example of a bacterium in which such a gene has been amplified includes, a *Bacillus* bacterium in which the *rhtA* gene is amplified (Japanese Patent Laid-open No. 2003-219876).

The *purR, deoD, pupG, purA* and *guaB* genes to be disrupted as described above, and the *prs* gene of which expression is to be enhanced may be variant DNAs encoding proteins having amino acid sequences of SEQ ID NOS: 6, 8, 10, 12, 14, 16 and 4 which include substitutions, deletions, insertions, additions or inversions of one to 10 amino acid residues, respectively, and having activity of the purine repressor, purine nucleoside phosphorylase, succinyl-AMP synthase, IMP dehydrogenase or phosphoribosyl pyrophosphate synthetase, respectively.

Such change of amino acid sequences as described above is usually conservative change which maintains activities of proteins. Examples of conservative amino acid substitution include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val.

Specific examples of other conservative variants of the *purR, deoD, pupG, purA, guaB* and *fbp* genes and the *prs* gene described herein include DNAs showing a homology of, for example, 70% or more, preferably 80% or more, more preferably 90% or more, particularly preferably 95% or more, to DNAs having the nucleotide sequences of SEQ ID NOS: 5, 7, 9, 11, 13, 15 and 3, respectively. More specifically, the examples of the conservative variants described herein include DNAs that are able to hybridize with DNAs having nucleotide sequences complementary to the nucleotide sequences of SEQ ID NOS: 5, 7, 9, 11, 13, 15 and 3 under stringent conditions. An example of the stringent conditions includes conditions of washing at 60°C and salt concentrations of 1×SSC, 0.1% SDS, preferably 0.1×SSC, 0.1% SDS, once or more times, preferably twice or three times.

Homology of DNAs can be evaluated by BLAST search, FASTA search, the calculation method of Crustal W, and so forth.

BLAST (basic local alignment search tool) is a heuristic search algorithm used by the programs blastp, blastn, blastx, megablast, tblastn and tblastx, and the results obtained by these programs are considered significant on the basis of use of the statistical method of Karlin, Samuel, and Stephen F. Altschul ("Methods for assessing the statistical significance of molecular sequence features by using general scoring schemes", Proc. Natl. Acad. Sci. USA, 1990, 87:2264-68; "Applications and statistics for multiple high-scoring segments in molecular sequences", Proc. Natl. Acad. Sci. USA, 1993, 90:5873-7). The FASTA search method was described by W.R. Pearson ("Rapid and Sensitive Sequence Comparison with FASTP and FASTA", Methods in Enzymology, 1990 183:63-98). The Clustal W method is described by Thompson J.D., Higgins D.G., and Gibson T.J. ("CLUSTAL W: Improving the sensitivity of progressive multiple sequence alignment through sequence weighting, position-specific gap penalties and weight matrix choice", Nucleic Acids Res., 1994, 22:4673-4680).

Moreover, DNA used for preparation of a disrupted-type gene may also be a conservative variant of *purR, deoD, pupG, purA* or *guaB* gene.

Incorporation of a target gene into the chromosomal DNA of *Bacillus* bacterium can be performed in the same manner as that for the gene encoding fructose bisphosphatase described later.

### (II) Modification for decreasing enzymatic activity of fructose bisphosphatase

The *Bacillus* bacterium used in the method of present invention can be obtained by modifying a strain having the ability to produce a purine-derived substance such as those described above so that the enzymatic activity of fructose bisphosphatase is decreased. The order of modification is not limited, and after modifying a bacterium so that the enzymatic activity of fructose bisphosphatase thereof is decreased, purine nucleotide producing ability may be imparted to the bacterium.

Fructose bisphosphatase is an enzyme which catalyzes a reaction generating fructose-6-phosphate from fructose-1,6-bisphosphate, and this reaction is one of the reactions of the glyconeogenesis pathway. The "glyconeogenesis pathway" means a pathway in which intracellular oxaloacetic acid is converted into phosphoenolpyruvic acid by decarboxylation catalyzed by phosphoenolpyruvate carboxykinase (EC: 4.1.1.49) and phosphorylation, phosphoenolpyruvic acid is converted into fructose-1,6-bisphosphate by the reverse reactions of glycolytic enzymes, fructose-1,6-bisphosphate is further converted into fructose-6-phosphate by fructose bisphosphatase (EC: 3.1.3.11), and glucose is biosynthesized from fructose-6-phosphate by glucose-6-phosphate isomerase and glucose-6-phosphatase (EC: 3.1.3.9).

The enzymatic activity of fructose bisphosphatase can be measured by the following method. For example, it can be measured by converting generated fructose-6-phosphate into NADPH by phosphoglucoisomerase and glucose-6-phosphate dehydrogenase, and measuring NADPH.

Such modification that the enzymatic activity of fructose bisphosphatase is decreased can be attained by, for example, as explained above for disruption of the *purR* gene, substituting a corresponding gene which does not function normally (e.g., disrupted-type gene obtained by inserting a marker gene such as drug resistance gene into the fructose bisphosphatase gene) for the fructose bisphosphatase gene on the chromosome by homologous recombination. Furthermore, as described for the *purR* gene, a mutation for reducing intracellular enzymatic activity of fructose bisphosphatase may be introduced into the fructose bisphosphatase gene on the chromosome by conventional mutagenesis methods.

An example of fructose bisphosphatase of *Bacillus subtilis* includes a protein consisting of 671 amino acids shown as SEQ ID NO: 2, and a gene encoding the protein, preferably a gene having the nucleotide sequence of SEQ ID NO: 1 (fbp gene, nucleotide numbers 4127053 to 4129065 of Genbank Accession No. NC_000964), can be used for the modification. The *fbp* gene locates at about 323° on the *Bacillus subtilis* chromosome.

Examples of DNA encoding a protein substantially identical to fructose bisphosphatase include, specifically, a DNA encoding a protein showing a homology of 50% or more, preferably 70% or more, more preferably 80% more, particularly preferably 90% or more, most preferably 95% or more, to the amino acid sequence shown in SEQ ID NO: 2, and having the enzymatic activity of fructose bisphosphatase.

The gene encoding fructose bisphosphatase may also be a conservative variant of the *fbp* gene like the aforementioned genes. Specifically, examples include a DNA encoding a protein having the amino acid sequence of SEQ ID NO: 2 including substitutions, deletions, insertions, additions or inversions of one to 10 amino acid residues and having fructose bisphosphatase activity. A DNA encoding a protein showing a homology of 50% or more, preferably 70% or more, more preferably 80% more, particularly preferably 90% or more, most preferably 95% or more, to the amino acid sequence shown in SEQ ID NO: 2, and having the enzymatic activity of fructose bisphosphatase is disclosed herein. More specifically, examples include a DNA which is able to hybridize with a DNA having the nucleotide sequence of SEQ ID NO: 1 under stringent conditions is disclosed herein. The stringent conditions include conditions of washing at 60°C and salt concentrations of 1×SSC, 0.1% SDS, preferably 60°C, 0.1×SSC, 0.1% SDS, once or more times, preferably twice or three times.

Such a DNA encoding a protein substantially identical to fructose bisphosphatase as described above can be obtained by, for example, modifying a nucleotide sequence encoding such an enzyme so that the amino acid residue of a specific part is substituted, deleted, inserted, added or inverted by the site-specific mutagenesis. Such a modified DNA as described above may also be obtained by a conventionally known mutagenesis treatment. Examples of the mutagenesis treatment include an in vitro treatment of DNA before mutagenesis treatment with hydroxylamine, and a treatment of a microorganism such as *Escherichia* bacterium containing DNA before mutagenesis treatment with ultraviolet irradiation or a mutagen used for conventional mutagenesis treatment such as N-methyl-N'-nitro-N-nitrosoguanidine (NTG) or nitrous acid.

The target gene can be obtained by, for example, PCR method (polymerase chain reaction, White, T.J. et al., Trends Genet., 1989, 5, 185-189) using a chromosomal DNA of a *Bacillus* bacterium as a template and oligonucleotides prepared based on nucleotide sequence of target gene as primers. The chromosomal DNA can be prepared from a bacterium serving as a DNA donor by, for example, the method of Saito and Miura (H. Saito and K. Miura, Biochem. Biophys. Acta, 1963, 72, 619-629; Text for Bioengineering Experiments, Edited by the Society for Bioscience and Bioengineering, Japan, pp.97-98, Baifukan, 1992) or the like. The primers for PCR can be prepared based on a known gene sequence of a *Bacillus* bacterium, or based on information on a region conserved among genes of other bacteria of which sequences are known.

Examples of the vector for incorporating a target gene into a chromosomal DNA of *Bacillus* bacterium include a vector having a temperature sensitive replication origin, such as pHV1248 (Prtit, M.-A., et. al., J. Bacteriol., 1990, 172, 6736-6740), vectors for *E*. *coli* such as pHSG398 (Takara Shuzo) and pBluescript SK- (Stratagene), and so forth.

In order to ligate an objective gene and a vector carrying a marker which functions in *Bacillus* bacteria to prepare a recombinant DNA, the vector is digested with a restriction enzyme matching the end of the objective gene. The ligation is usually performed by using a ligase such as T4 DNA ligase.

To introduce a recombinant DNA prepared as described above into a *Bacillus* bacterium, any of known transformation methods that have hitherto been reported can be employed. Examples include, for instance, a method of preparing competent cells from cells which are at the growth phase followed by introducing the DNA thereinto,

(Dubunau D. and Davidoff-Abelson, R., J. Mol. Biol., 1971, 56, 209-221) and a method of making host cells into protoplasts or spheroplasts, which can easily take up recombinant DNA, followed by introducing the recombinant DNA into the DNA-acceptor cells (Chang, S. and Choen, S.N., Molec. Gen. Genet., 1979, 168, 111-115).

### <2> Method for producing a purine-derived substance

The *Bacillus* bacterium used for the present method efficiently produces a purine-derived substance. Therefore, by culturing such a *Bacillus bacterium* in an appropriate medium, a purine-derived substance, such as purine nucleoside and purine nucleotide can be produced and accumulated in cells of the bacterium or the medium.

As for the medium used for the present method culture can be performed in a conventional manner using a conventional medium containing a carbon source, nitrogen source and mineral salts as well as organic trace nutrients such as amino acids and vitamins, as required. Either a synthetic medium or a natural medium may be used. Any kinds of carbon source and nitrogen source may be used so long as they can be utilized by a strain to be cultured.

As the carbon source, sugars such as glucose, fructose, sucrose, maltose, mannose, galactose, arabinose, xylose, trehalose, ribose, starch hydrolysates and molasses, and alcohols such as glycerol and mannitol are used, and organic acids such as gluconic acid, acetic acid, citric acid, maleic acid, fumaric acid and succinic acid can also be used independently or in combination with other carbon sources.

As the nitrogen source, ammonia, ammonium salts such as ammonium sulfate, ammonium carbonate, ammonium chloride, ammonium phosphate and ammonium acetate, nitric acid salts, organic nitrogen such as soybean hydrolysate, and so forth are used.

As the organic trace nutrients, amino acids, vitamins, fatty acids, nucleic acids, those containing these substances such as peptone, casamino acid, yeast extract and soybean protein decomposition product and so forth are used. When an auxotrophic mutant strain that requires an amino acid or the like for growth is used, it is necessary to supplement the required nutrient.

As the mineral salts, phosphoric acid salts, magnesium salts, calcium salts, iron salts, manganese salts and so forth are used.

Although the culture conditions may vary depending on the type of *Bacillus* bacterium to be used, *Bacillus subtilis,* for example, is cultured as aeration culture, while the fermentation temperature is controlled to be 20 to 50°C, and pH to be 4 to 9. When pH falls during the culture, the medium is neutralized with an alkali such as ammonia gas. A purine nucleoside is accumulated in the medium after about 40 hours to 3 days of culture in such a manner as described above.

After completion of the culture, inosine accumulated in the medium may be collected in a conventional manner. For example, it can be isolated by precipitation, ion exchange chromatography and so forth.

Furthermore, if the microorganism used for the present method lacks a gene encoding a nucleosidase or nucleotidase, a corresponding nucleoside or nucleotide can be accumulated. Furthermore, if inosine auxotrophy is imparted, a precursor or relevant substances involved in the biosynthesis pathway thereof can be accumulated.

Furthermore, by reacting inosine or guanosine prepared by the method of the present invention with purine nucleoside phosphorylase or phosphoribosyltransferase, 5'-inosinic acid or 5'-guanylic acid can be obtained.

Moreover, it is also possible to phosphorylate the purine nucleoside produced using the microorganism of the present invention by reacting phosphotransferase on the purine nucleoside to produce a purine nucleotide (nucleoside 5'-phosphoric acid ester) (Japanese Patent Laid-open No. 2000-295996). For example, the method for producing a purine nucleotide using an *Escherichia* bacterium into which a gene encoding inosine guanosine kinase of *Escherichia coli* is introduced (WO91/08286), and the method for producing a purine nucleotide using *Corynebacterium ammoniagenes* into which a gene encoding inosine guanosine kinase of *Exiguobacterium acetylicum* is introduced (WO96/30501) can be used.

Moreover, it is also possible to produce a purine nucleotide (nucleoside 5'-phosphoric acid ester) by reacting the purine nucleoside produced by using the microorganism described herein with a phosphate donor selected from the group consisting of polyphosphoric acid, phenyl phosphate and carbamyl phosphate, and a microorganism having an ability to produce a nucleoside 5'-phosphoric acid ester or acid phosphatase (EC 3.1.3.2). Although the microorganism having an ability to produce a nucleoside 5'-phosphoric acid ester is not particularly limited so long as a microorganism having an ability to convert a purine nucleoside into a purine nucleotide is chosen, examples include, for example, the microorganism disclosed in International Patent Publication WO96/37603.

Moreover, *Escherichia* blattae JCM 1650, *Serratia ficaria* ATCC 33105, *Klebsiella planticola* IFO 14939 (ATCC 33531), *Klebsiella pneumoniae* IFO 3318 (ATCC 8724), *Klebsiella terrigena* IFO 14941 (ATCC 33257), *Morganella morganii* IFO 3168, *Enterobacter aerogenes* IFO 12010, *Enterobacter aerogenes* IFO 13534 (ATCC 13048), *Chromobacterium fluviatile* IAM 13652, *Chromobacterium violaceum* IFO 12614, *Cedecea lapagei* JCM 1684, *Cedecea davisiae* JCM 1685, *Cedecea neteri* JCM 5909, and so forth disclosed in Japanese Patent Laid-open No. 07-231793 can also be used.

As the acid phosphatase, for example, the acid phosphatase disclosed in Japanese Patent Laid-open No. 2002-000289 can be used, and an acid phosphatase of which affinity to a nucleoside is increased (Japanese Patent Laid-open No. 10-201481), a mutant acid phosphatase of which nucleotidase activity is decreased (WO96/37603), a mutant acid phosphatase of which phosphoric acid ester hydrolysis activity is decreased (Japanese Patent Laid-open No. 2001-245676) and so forth can be more preferably used.

It is also possible to obtain a purine nucleotide by chemically phosphorylating a purine nucleoside produced using the microorganism described herein (Bulletin of the Chemical Society of Japan, 42, 3505). Moreover, a method of obtaining GMP by coupling the microorganism having an XMP-producing ability described herein and XMP aminase activity using the ATP-regenerating system of a microorganism, and a method of obtaining IMP by coupling inosine kinase (Biosci. Biotech. Biochem., 51, 840 (1997); Japanese Patent Laid-open No. 63-230094) can also be used.

Inosine, guanosine or purine nucleoside prepared by the method of the present invention used for the aforementioned preparation of purine nucleotide may be a purified product, or purine nucleoside fermentation broth, or a crude product containing a purine nucleoside.

### Examples

Hereafter, the present invention will be more specifically explained with reference to examples.

### Example 1

### <Construction of bacterial strain deficient in the pupG and deoD genes encoding purine nucleoside phosphorylases>

A strain deficient in the purine nucleoside phosphorylase gene (*deoD*) was constructed from the recombinant strain KMBS310 as described below. The KMBS310 strain (Japanese Patent Application No. 2005-280186), which is derived from *Bacillus subtilis* (*B*. *subtilis* 168 Marburg strain, ATCC 6051), is deficient in the purine operon repressor gene (*purR*), succinyl-AMP synthase gene (*purA*) and purine nucleoside phosphorylase gene (*pupG*), and has attenuated IMP dehydrogenase gene (*guaB*), wherein the purine operon promoter region and SD sequence of the PRPP synthetase gene (*prs*) was modified. Expression of the purine operon and the PRPP synthetase gene was enhanced by the modifications of the promoter region and the SD sequence, respectively.

A genome DNA prepared from the KMBS16 strain (purR::spc purA::erm deoD::kan, Japanese Patent Laid-open No. 2004-242610) by the method of Fouet and Sonenshein (J. Bacteriol., 1990, 172, 835-844) was used to transform competent cells of the *B. subtilis* 168 Marburg strain prepared by the method of Dubnau and Davidoff-Abelson (J. Mol. Biol., 1971, 56, 209-221), and colonies grown on an LB agar plate containing 5 µg/ml of kanamycin were obtained. Colonies which did not became spectinomycin-resistant nor erythromycin-resistant were selected from the obtained colonies, and one strain among them was designated KMBS5 (deoD::kan).

A genomic DNA prepared from KMBS5 by the method of Fouet and Sonenshein (J. Bacteriol., 1990, 172, 835-844) was used to transform competent cells of the KMBS310 strain prepared by the method of Dubnau and Davidoff-Abelson (J. Mol. Biol., 1971, 56, 209-221), and colonies grown on an LB agar plate containing 5 µg/ml of kanamycin and 20 µg/ml of guanine were obtained. Several colonies were selected from the colonies obtained as described above, and one of transformants for which it could be confirmed that deoD::kan substituted for the wild-type *deoD* gene, and all the mutations derived from KMBS310 were not replaced with wild-type sequences was designated KMBS321.

### Example 2

### <Construction of bacterial strain deficient in fbp gene encoding fructose bisphosphatase, culture and evaluation thereof>

### (1) Preparation of fbp gene-deficient strain

A strain deficient in the fructose bisphosphatase gene (*fbp*) was consrtucted from the aforementioned recombinant strain KMBS321 as described below. The KMBS321 strain, which is derived from *Bacillus subtilis* (*B*. *subtilis* 168 Marburg strain, ATCC 6051), is deficient in the purine operon repressor gene (*purR*), succinyl-AMP synthase gene (*purA*) and purine nucleoside phosphorylase gene (*pupG*), and has attenuated IMP dehydrogenase gene (*guaB*), wherein purine operon promoter region and SD sequence of the PRPP synthetase gene (*prs*) is modified.

### (i) Amplification of fbp upstream region by PCR

28-mer and 50-mer PCR primers having the following nucleotide sequences were prepared based on the information of a gene data bank (GenBank Accession Nos. NC_000964 and V01277).
TTCCCTTAGGGTTATTTTCGTTTCAAAA (SEQ ID NO: 17) cgtttgttgaactaatgggtgctTTTATGAGCATGTGCATGATAAGGTGA (SEQ ID NO: 18, the nucleotides indicated with small letters correspond to the promoter upstream region of the chloramphenicol resistance gene (cat) cloned in pC194)

PCR (98°C for 10 second, 55°C for 30 seconds, 72°C for 1.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a *fbp* gene 5' end region and about 1350 bp upstream region.

### (ii) Amplification of fbp downstream region by PCR

50-mer and 27-mer PCR primers having the following nucleotide sequences were prepared based on the information of a gene data bank (GenBank Accession Nos. NC_000964 and V01277).
acagctccagatccatatccttcttTTTTAGAGAGTTTGCGGGAGTATCG (SEQ ID NO: 19, the nucleotides indicated with small letters correspond to a downstream region of structural gene of the chloramphenicol resistance gene (cat) cloned in pC194)
TAAAGGTTTTTCGGGATAAGATTGAAA (SEQ ID NO: 20)

PCR (98°C for 10 second, 55°C for 30 seconds, 72°C for 1.5 minutes, 30 cycles, Gene Amp PCR System Model 9600, Perkin-Elmer) was performed using the chromosomal DNA of the *B. subtilis* 168 Marburg strain as a template and the aforementioned primers to obtain an amplification fragment containing a *fbp* gene 3' end region and about 1770 bp downstream region.

### (iii) Amplification of cat gene by PCR

50-mer PCR primers having the following nucleotide sequences were prepared based on the information of a gene data bank (GenBank Accession Nos. V01277 and NC_000964).
tcaccttatcatgcacatgctcataaaAGCACCCATTAGTTCAACAAACG (SEQ ID NO: 21, the nucleotides indicated with small letters correspond to the sequence of 5' end region of the *fbp* gene, and they were designed so as to be complementary to the 3' end region of SEQ ID NO: 18)
cgatactcccgcaaactctctaaaaAAGAAGGATATGGATCTGGAGCTGT (SEQ ID NO: 22, the nucleotides indicated with small letters correspond to the sequence of 3' end region of the *fbp* gene, and they were designed so as to be complementary to the 3' end region of SEQ ID NO: 19)

PCR (98°C for 10 second, 55°C for 30 seconds, 72°C for 1.5 minutes, 30 cycles, Gene Amp PCR System Model 9600, Perkin-Elmer) was performed using the plasmid pC194 carrying the chloramphenicol resistance gene (cat) as a template and the aforementioned primers to obtain an amplification fragment of about 980 bp containing the *cat* gene.

### (iv) Amplification of fragment comprising fbp region inserted with cat gene by recombinant PCR

The DNA fragments amplified in (i) to (iii) described above were purified using MicroSpin Column S-400 (Amersham Pharmacia Biotech), and then a mixture of them in appropriate amounts was used as a template together with the sequences of SEQ ID NOS: 17 and 20 to perform PCR (98°C for 10 second, 55°C for 30 seconds, 72°C for 4.5 minute, 30 cycles, Gene Amp PCR System Model 9600, Perkin-Elmer) and thereby obtain a fragment comprising the *fbp* region inserted with the *cat* gene.

### (v) Preparation of fbp-disrupted inosine-producing strain

The DNA fragment comprising the *fbp* region into which the *cat* gene (fbp::*cat*) obtained in (iv) had been inserted was subjected to agarose gel electrophoresis, and the target fragment was extracted from the gel. The DNA fragment purified as described above was used to transform competent cells of the *B. subtilis* KMBS321 strain prepared by the method of Dubnau and Davidoff-Abelson (J. Mol. Biol., 1971, 56, 209-221), and colonies grown on an LB agar plate containing 2.5 µg/ml of chloramphenicol and 20 µg/ml of guanine were obtained. Chromosomal DNAs were prepared from these colonies, strains in which *fbp* region on a chromosome was replaced with the *fbp* region of which internal sequence was replaced with the chloramphenicol resistance gene (fbp::cat) by double recombination were identified by the PCR method described in (iv), and one of these strains was designated TABS133.

### (2) Production of purine nucleoside by inosine-producing strain deficient in fbp gene.

The *fbp* gene-deficient strain TABS133 and a control strain KMBS321 were each uniformly applied on an LB medium plate containing 20 mg/L of guanine, and cultured overnight at 34°C. The cells corresponding to 1/8 of the plate were inoculated into 20 ml of a fermentation medium contained in a 500-ml volume Sakaguchi flask, then 50 g/L of calcium carbonate was added to the medium, and the cells were cultured at 34°C with shaking. Seventy two hours after the start of the culture, the medium was sampled, and amounts of inosine and hypoxanthine contained in the medium were measured by known methods. The accumulated inosine amount observed with the *fbp* gene-deficient strain TABS133 was higher than that observed with the KMBS321 strain as a control strain.

### [Composition of fermentation medium]

| | |
|---|---|
| Glucose | 30 g/L |
| KH₂PO₄ | 1 g/L |
| NH₄Cl | 32 g/L |
| Mameno (T-N) * | 1.35 g/L |
| DL-Methionine | 0.4 g/L |
| L-Tryptophan | 0.02 g/L |
| Adenine | 0.1 g/L |
| Guanosine | 0.075 g/L |
| MgSO₄ | 0.4 g/L |
| FeSO₄ | 0.01 g/L |
| MnSO₄ | 0.01 g/L |
| Adekanol (antifoam) (adjusted to pH 7.0 with KOH) | 0.5 ml/L |
| Calcium Carbonate | 50 g/L |

| | |
|---|---|
| *: Soybean protein hydrolysate | |

**Table 1**

| *B. subtilis* strains | inosine (g/L) |
|---|---|
| KMBS321 | 5.3 |
| TABS133 | 5.8 |

### [Explanation of Sequence Listing]

SEQ ID NO: 1: Nucleotide sequence of *fbp* gene
SEQ ID NO: 2: Amino acid sequence of fructose bisphosphatase
SEQ ID NO: 3: Nucleotide sequence of *prs* gene
SEQ ID NO: 4: Amino acid sequence of phosphoribosyl pyrophosphate synthetase
SEQ ID NO: 5: Nucleotide sequence of *purR* gene
SEQ ID NO: 6: Amino acid sequence of purine repressor
SEQ ID NO: 7: Nucleotide sequence of *deoD* gene
SEQ ID NO: 8: Amino acid sequence of *deoD* gene product (purine nucleoside phosphorylase)
SEQ ID NO: 9: Nucleotide sequence of *pupG* gene
SEQ ID NO: 10: Amino acid sequence of *pupG* gene product (purine nucleoside phosphorylase)
SEQ ID NO: 11: Nucleotide sequence of *purA* gene
SEQ ID NO: 12: Amino acid sequence of succinyl-AMP synthase
SEQ ID NO: 13: Nucleotide sequence of *guaB* gene
SEQ ID NO: 14: Amino acid sequence of IMP dehydrogenase
SEQ ID NO: 15: Primer for *purR* gene amplification
SEQ ID NO: 16: Primer for *purR* gene amplification
SEQ ID NO: 17: Primer for *fbp* gene upstream region amplification
SEQ ID NO: 18: Primer for *fbp* gene upstream region amplification
SEQ ID NO: 19: Primer for *fbp* gene downstream region amplification
SEQ ID NO: 20: Primer for *fbp* gene downstream region amplification
SEQ ID NO: 21: Primer for *cat* gene amplification
SEQ ID NO: 22: Primer for *cat* gene amplification

### Industrial Applicability

By using the method of the present invention, production efficiency of a purine nucleoside and/or a purine nucleotide can be improved.

## Claims

1. A method for producing a purine-derived substance, which comprises
culturing a bacterium belonging to the genus *Bacillus* which has an ability to produce a purine-derived substance in a medium to cause accumulation of the purine-derived substance in cells of the bacterium or the medium, and
collecting the purine-derived substance from the cells or medium,
wherein the bacterium has been modified so that enzymatic activity of fructose bisphosphatase is decreased compared to an unmodified strain by disruption of a gene encoding fructose bisphosphatase, or decreasing expression amount of the gene encoding fructose bisphosphatase,
wherein the gene encoding fructose bisphosphatase is a gene encoding a protein of the following (A) or (B):
(A) a protein comprising the amino acid sequence of SEQ ID NO: 2,
(B) a protein comprising the amino acid sequence of SEQ ID NO: 2 which includes substitutions, deletions, insertions, additions or inversions of 1 to 10 amino acid residues and has fructose bisphosphatase activity, and
wherein the purine-derived substance is a purine nucleoside selected from the group consisting of inosine, xanthosine, guanosine and adenosine or a purine nucleotide selected from the group consisting of inosinic acid, xanthylic acid, guanylic acid and adenylic acid.

2. The method according to claim 1, wherein the bacterium has been further modified so that phosphoribosyl pyrophosphate synthetase activity is increased compared to an unmodified strain by increasing expression of a gene encoding the phosphoribosyl pyrophosphate synthetase in the bacterium by using a plasmid containing the gene or integrating the gene into the chromosome of the bacterium, and wherein the gene encoding phosphoribosyl pyrophosphate synthetase is a gene encoding a protein of the following (A) or (B) :
(A) a protein comprising the amino acid sequence of SEQ ID NO: 4,
(B) a protein comprising the amino acid sequence of SEQ ID NO: 4 which includes substitutions, deletions, insertions, additions or inversions of 1 to 10 amino acid residues and has phosphoribosyl pyrophosphate synthetase activity.

3. The method according to claim 1 or 2, wherein the bacterium has been further modified so that expression amount of the purine operon is increased compared to an unmodified strain by increasing expression of genes of the purine operon in the bacterium by a method of using a plasmid containing the genes or integrating the genes into the chromosome of the bacterium, by replacing the native promoter of the purine operon with a stronger promoter, by replacing the -35 or -10 region of the native promoter of the purine operon with a consensus sequence, by disrupting a gene encoding a purine repressor, by decreasing the expression amount of the gene encoding the purine repressor, or deleting the attenuator sequence of the purine operon, and
wherein the gene encoding purine repressor is a gene encoding a protein of the following (A) or (B):
(A) a protein comprising the amino acid sequence of SEQ ID NO: 6,
(B) a protein comprising the amino acid sequence of SEQ ID NO: 6 which includes substitutions, deletions, insertions, additions or inversions of 1 to 10 amino acid residues and has purine repressor activity.

4. The method according to any one of claims 1 to 3, wherein the bacterium has been further modified so that purine nucleoside phosphorylase activity is decreased compared to an unmodified strain by disrupting a gene encoding purine nucleoside phosphorylase or decreasing expression amount of the gene encoding purine nucleoside phosphorylase, and wherein the gene encoding purine nucleoside phosphorylase is a gene encoding a protein of the following (A) or (B):
(A) a protein comprising the amino acid sequence of SEQ ID NO: 8 or 10,
(B) a protein comprising the amino acid sequence of SEQ ID NO: 8 or 10 which includes substitutions, deletions, insertions, additions or inversions of 1 to 10 amino acid residues and has purine nucleoside phosphorylase activity.

5. The method according to any one of claims 1 to 4, wherein the bacterium has been further modified so that IMP dehydrogenase activity is decreased compared to an unmodified strain by decreasing expression amount of the gene encoding IMP dehydrogenase, and wherein the gene encoding IMP dehydrogenase is a gene encoding a protein of the following (A) or (B):
(A) a protein comprising the amino acid sequence of SEQ ID NO: 14,
(B) a protein comprising the amino acid sequence of SEQ ID NO: 14 which includes substitutions, deletions, insertions, additions or inversions of 1 to 10 amino acid residues and has IMP dehydrogenase activity.

6. The method according to any one of claims 1 to 5,
wherein the bacterium is *Bacillus subtilis.*

7. A method for producing a purine nucleotide, which comprises
producing a purine nucleoside by the method according to any one of claims 1 to 6,
reacting the purine nucleoside with a phosphate donor selected from the group consisting of polyphosphoric acid, phenyl phosphate and carbamyl phosphate, and a microorganism having an ability to produce a nucleoside-5'-phosphoric acid ester or acid phosphatase to produce a purine nucleotide, and
collecting the purine nucleotide.

## Patentansprüche

1. Verfahren zum Herstellen einer von Purin abgeleiteten Substanz, welches
das Kultivieren eines Bakteriums der Gattung *Bacillus,* das die Fähigkeit zur Produktion einer von Purin abgeleiteten Substanz in einem Medium hat, wobei die Anhäufung der von Purin abgeleiteten Substanz in Zellen des Bakteriums oder dem Medium bewirkt wird, und
das Gewinnen der von Purin abgeleiteten Substanz aus den Zellen oder dem Medium umfasst, wobei das Bakterium so modifiziert worden ist, dass die enzymatische Aktivität von Fructosebisphosphatase im Vergleich zu einem unmodifizierten Stamm durch Unterbrechung eines für Fructosebisphosphatase kodierenden Gens oder durch Senken der Expressionsmenge des für Fructosebisphosphatase kodierenden Gens verringert ist,
wobei das für Fructosebisphosphatase kodierende Gen ein Gen ist, das für ein Protein (A) oder (B) kodiert:
(A) ein Protein, das die Aminosäuresequenz der SEQ ID NO: 2 umfasst,
(B) ein Protein, das die Aminosäuresequenz der SEQ ID NO: 2 enthält, welches Substitutionen, Deletionen, Insertionen, Additionen oder Inversionen von 1 bis 10 Aminosäureresten umfasst und Fructosebisphosphatase-Aktivität hat, und
wobei die von Purin abgeleitete Substanz ein Purinnukleosid, das aus der Gruppe ausgewählt ist, die aus Inosin, Xanthosin, Guanosin und Adenosyn besteht, oder ein Purinnukleotid ist, das aus der Gruppe ausgewählt ist, die aus Inosinsäure, Xanthylsäure, Guanylsäure und Adenylsäure besteht.

2. Verfahren nach Anspruch 1, wobei das Bakterium außerdem so modifiziert worden ist, dass die Phosphoribosylpyrophosphatsynthetase-Aktivität im Vergleich zu einem unmodifizierten Stamm durch Erhöhen der Expression eines für Phosphoribosylpyrophosphatsynthetase kodierenden Gens in dem Bakterium erhöht ist, indem ein das Gen enthaltendes Plasmid verwendet wird oder das Gen in das Chromosom des Bakteriums integriert wird, und wobei das für Phosphoribosylpyrophosphatsynthetase kodierende Gen ein Gen ist, das für das folgende Protein (A) oder (B) kodiert:
(A) ein Protein, das die Aminosäuresequenz der SEQ ID NO: 4 umfasst,
(B) ein Protein, das die Aminosäuresequenz der SEQ ID NO: 4 enthält, welches Substitutionen, Deletionen, Insertionen, Additionen oder Inversionen von 1 bis 10 Aminosäureresten umfasst und Phosphoribosylpyrophosphatsynthetase-Aktivität hat.

3. Verfahren nach Anspruch 1 oder 2, wobei das Bakterium außerdem so modifiziert worden ist, dass die Expressionsmenge des Purin-Operons im Vergleich zu einem unmodifizierten Stamm erhöht ist, indem die Expression von Genen des Purin-Operons in dem Bakterium durch ein Verfahren erhöht wird, bei dem ein die Gene enthaltendes Plasmid verwendet wird, oder indem die Gene in das Chromosom des Bakteriums integriert werden, indem der native Promotor des Purin-Operons durch einen stärkeren Promotor ersetzt wird, indem die -35 oder -10-Region des nativen Promotors des Purin-Operons durch eine Konsensus-Sequenz ersetzt wird, indem ein für einen Purin-Repressor kodierendes Gen zerstört wird, indem die Expressionsmenge des für den Purin-Repressor kodierenden Gens verringert wird oder indem die Attenuatorsequenz des Purin-Operons entfernt wird, und
wobei das für den Purin-Repressor kodierende Gen ein Gen ist, das für das folgende Protein (A) oder (B) kodiert:
(A) ein Protein, das die Aminosäuresequenz der SEQ ID NO: 6 enthält,
(B) ein Protein, das die Aminosäuresequenz der SEQ ID NO: 6 enthält, welches Substitutionen, Deletionen, Insertionen, Additionen oder Inversionen von 1 bis 10 Aminosäureresten umfasst und Purinrepressor-Aktivität aufweist.

4. Verfahren nach einem der Ansprüche 1 bis 3, wobei das Bakterium außerdem so modifiziert worden ist, dass die Purinnukleosidphosphorylase-Aktivität im Vergleich zu einem unmodifizierten Stamm verringert ist, indem ein für Purinnukleosidphosphorylase kodierendes Gen zerstört wird oder die Expressionsmenge des für Purinnukleosidphosphorylase kodierenden Gens verringert wird, und wobei das für Purinnukleosidphosphorylase kodierende Gen ein Gen ist, das für das folgende Protein (A) oder (B) kodiert:
(A) ein Protein, das die Aminosäuresequenz der SEQ ID NO: 8 oder 10 enthält,
(B) ein Protein, das die Aminosäuresequenz der SEQ ID NO: 8 oder 10 enthält, welches Substitutionen, Deletionen, Insertionen, Additionen oder Inversionen von 1 bis 10 Aminosäureresten enthält und Purinnukleosidphosphorylase-Aktivität aufweist.

5. Verfahren nach einem der Ansprüche 1 bis 4, wobei das Bakterium außerdem so modifiziert worden ist, dass die IMP-Dehydrogenaseaktivität im Vergleich zu einem unmodifizierten Stamm verringert ist, indem die Expressionsmenge des für IMP-Dehydrogenase kodierenden Gens verringert ist, und wobei das für IMP-Dehydrogenase kodierende Gen ein Gen ist, das für das folgende Protein (A) oder (B) kodiert:
(A) ein Protein, das die Aminosäuresequenz der SEQ ID NO: 14 enthält,
(B) ein Protein, das die Aminosäuresequenz der SEQ ID NO: 14 enthält, welches Substitutionen, Deletionen, Insertionen, Additionen oder Inversionen von 1 bis 10 Aminosäureresten umfasst und IMP-Dehydrogenaseaktivität aufweist.

6. Verfahren nach einem der Ansprüche 1 bis 5, wobei das Bakterium *Bacillus subtilis* ist.

7. Verfahren zum Herstellen eines Purin-Nukleotids, welches das Produzieren eines Purin-Nukleosids durch das Verfahren nach einem der Ansprüche 1 bis 6, das Umsetzen des Purinnukleosids mit einem Phosphatdonor, der aus der aus Polyphosphorsäure, Phenylphosphat und Carbamylphosphat bestehenden Gruppe ausgewählt ist, und einem Mikroorganismus mit der Fähigkeit zur Produktion eines Nukleosid-5'-phosphorsäureesters oder saurer Phosphatase unter Produktion eines Purinnukleotids, und das Gewinnen des Purinnukleotids umfasst.

## Revendications

1. Procédé pour produire une substance dérivée d'une purine, qui consiste à
cultiver une bactérie appartenant au genre *Bacillus* qui possède une aptitude à produire une substance dérivée d'une purine dans un milieu afin de provoquer l'accumulation de la substance dérivée d'une purine dans les cellules de la bactérie ou le milieu, et
collecter la substance dérivée d'une purine à partir des cellules ou du milieu,
dans lequel la bactérie a été modifiée de sorte que l'activité enzymatique de la fructose bisphosphatase soit réduite par comparaison à une souche non modifiée par une perturbation d'un gène codant pour la fructose bisphosphatase, ou en réduisant la quantité d'expression du gène codant pour la fructose bisphosphatase,
dans lequel le gène codant pour la fructose bisphosphatase est un gène codant pour une protéine de (A) ou (B) ci-dessous :
(A) une protéine comprenant la séquence d'acides aminés de SEQ ID NO: 2,
(B) une protéine comprenant la séquence d'acides aminés de SEQ ID NO: 2 qui comprend des substitutions, délétions, insertions, additions ou inversions de 1 à 10 résidus d'acides aminés et possède une activité fructose bisphosphatase, et
dans lequel la substance dérivée d'une purine est un nucléoside purine sélectionné dans le groupe consistant en l'inosine, la xanthosine, la guanosine et l'adénosine, ou un nucléotide purine sélectionné dans le groupe consistant en l'acide inosinique, l'acide xanthylique, l'acide guanylique et l'acide adénylique.

2. Procédé selon la revendication 1, dans lequel la bactérie a en outre été modifiée de sorte que l'activité de la phosphoribosylpyrophosphate synthétase soit augmentée par comparaison à une souche non modifiée en augmentant l'expression d'un gène codant pour la phosphoribosylpyrophosphate synthétase chez la bactérie, en utilisant un plasmide contenant le gène ou en intégrant le gène dans le chromosome de la bactérie, et où le gène codant pour la phosphoribosylpyrophosphate synthétase est un gène qui code pour une protéine de (A) ou (B) ci-dessous :
(A) une protéine comprenant la séquence d'acides aminés de SEQ ID NO: 4,
(B) une protéine comprenant la séquence d'acides aminés de SEQ ID NO: 4 qui comprend des substitutions, délétions, insertions, additions ou inversions de 1 à 10 résidus d'acides aminés et possède une activité phosphoribosylpyrophosphate synthétase.

3. Procédé selon la revendication 1 ou 2, dans lequel la bactérie a en outre été modifiée de sorte que la quantité d'expression de l'opéron purine soit augmentée par comparaison à une souche non modifiée en augmentant l'expression des gènes de l'opéron purine chez la bactérie, par un procédé qui consiste à utiliser un plasmide contenant les gènes ou à intégrer les gènes dans le chromosome de la bactérie, en remplaçant le promoteur natif de l'opéron purine par un promoteur plus puissant, en remplaçant la région -35 ou -10 du promoteur natif de l'opéron purine par une séquence consensus, en perturbant un gène codant pour un répresseur purine, en réduisant la quantité d'expression du gène codant pour le répresseur purine, ou en supprimant la séquence d'atténuation de l'opéron purine, et
dans lequel le gène codant pour le répresseur purine est un gène qui code pour une protéine de (A) ou (B) ci-dessous :
(A) une protéine comprenant la séquence d'acides aminés de SEQ ID NO: 6,
(B) une protéine comprenant la séquence d'acides aminés de SEQ ID NO: 6 qui comprend des substitutions, délétions, insertions, additions ou
inversions de 1 à 10 résidus d'acides aminés et possède une activité de répresseur purine.

4. Procédé selon l'une quelconque des revendications 1 à 3, dans lequel la bactérie a en outre été modifiée de sorte que l'activité de la purine nucléoside phosphorylase soit réduite par comparaison à une souche non modifiée en perturbant un gène codant pour la purine nucléoside phosphorylase ou en réduisant la quantité d'expression du gène codant pour la purine nucléoside phosphorylase, et dans lequel le gène codant pour la purine nucléoside phosphorylase est un gène qui code pour une protéine de (A) ou (B) ci-dessous :
(A) une protéine comprenant la séquence d'acides aminés de SEQ ID NO: 8 ou 10,
(B) une protéine comprenant la séquence d'acides aminés de SEQ ID NO: 8 ou 10 qui comprend des substitutions, délétions, insertions, additions ou inversions de 1 à 10 résidus d'acides aminés et possède une activité purine nucléoside phosphorylase.

5. Procédé selon l'une quelconque des revendications 1 à 4, dans lequel la bactérie a en outre été modifiée de sorte que l'activité de l'IMP déshydrogénase soit réduite par comparaison à une souche non modifiée en réduisant la quantité d'expression du gène codant pour l'IMP déshydrogénase, et dans lequel le gène codant pour l'IMP déshydrogénase est un gène qui code pour une protéine de (A) ou (B) ci-dessous :
(A) une protéine comprenant la séquence d'acides aminés de SEQ ID NO: 14,
(B) une protéine comprenant la séquence d'acides aminés de SEQ ID NO: 14 qui comprend des substitutions, délétions, insertions, additions ou inversions de 1 à 10 résidus d'acides aminés et possède une activité IMP déshydrogénase.

6. Procédé selon l'une quelconque des revendications 1 à 5, dans lequel la bactérie est *Bacillus subtilis*

7. Procédé pour produire un nucléotide purine, qui consiste à
produire un nucléoside purine par le procédé selon l'une quelconque des revendications 1 à 6,
faire réagir le nucléoside purine avec un donneur de phosphate sélectionné dans le groupe consistant en l'acide polyphosphorique, le phénylphosphate et le carbamylphosphate, et un micro-organisme ayant une aptitude à produire un nucléoside-S'-ester d'acide phosphorique ou une phosphatase acide afin de produire un nucléotide purine, et
collecter le nucléotide purine.


### SEQUENCE LISTING

<110> Ajinomoto Co., Inc.
<120> Bacterium capable of producing purine substance, and process for production of purine substance
<130> EPA-64628
<150> JP2006-119315
   <151> 2006-04-24
<160> 22
<170> PatentIn version 3.3
<210> 1
   <211> 2013
   <212> DNA
   <213> Bacillus subtilis
<220>
   <221> CDS
   <222> (1)..(2013)
<400> 1
   atg ttt aaa aat aat gtc ata ctt tta aat tca cct tat cat gca cat      48
   Met Phe Lys Asn Asn Val Ile Leu Leu Asn Ser Pro Tyr His Ala His
   1               5                   10                  15
   gct cat aaa gag ggt ttt att cta aaa agg gga tgg acg gtt ttg gaa      96
   Ala His Lys Glu Gly Phe Ile Leu Lys Arg Gly Trp Thr Val Leu Glu
               20                  25                  30
   agc aag tac cta gat cta ctc gca caa aaa tac gat tgt gaa gaa aaa     144
   Ser Lys Tyr Leu Asp Leu Leu Ala Gln Lys Tyr Asp Cys Glu Glu Lys
           35                  40                  45
   gtg gta aca gaa atc atc aat ttg aaa gcg ata ttg aac ctg cca aaa     192
   Val Val Thr Glu Ile Ile Asn Leu Lys Ala Ile Leu Asn Leu Pro Lys
       50                  55                  60
   ggc acc gag cat ttt gtc agt gat ctg cac gga gag tat cag gca ttc     240
   Gly Thr Glu His Phe Val Ser Asp Leu His Gly Glu Tyr Gln Ala Phe
   65                  70                  75                  80
   cag cac gtg ttg cgc aat ggt tca gga cga gtc aaa gag aag ata cgc     288
   Gln His Val Leu Arg Asn Gly Ser Gly Arg Val Lys Glu Lys Ile Arg
                   85                  90                  95
   gac atc ttc agc ggt gtc att tac gat aga gaa att gat gaa tta gca     336
   Asp Ile Phe Ser Gly Val Ile Tyr Asp Arg Glu Ile Asp Glu Leu Ala
               100                 105                 110
   gca ttg gtc tat tat ccg gaa gac aaa ctg aaa tta atc aaa cat gac     384
   Ala Leu Val Tyr Tyr Pro Glu Asp Lys Leu Lys Leu Ile Lys His Asp
           115                 120                 125
   ttt gat gcg aaa gaa gcg tta aac gag tgg tat aaa gaa acg att cat     432
   Phe Asp Ala Lys Glu Ala Leu Asn Glu Trp Tyr Lys Glu Thr Ile His
       130                 135                 140
   cga atg att aag ctc gtt tca tat tgc tcc tct aag tat acc cgc tcc     480
   Arg Met Ile Lys Leu Val Ser Tyr Cys Ser Ser Lys Tyr Thr Arg Ser
   145                 150                 155                 160
   aaa tta cgc aaa gca ctg cct gcc caa ttt gct tat att acg gag gag     528
   Lys Leu Arg Lys Ala Leu Pro Ala Gln Phe Ala Tyr Ile Thr Glu Glu
                   165                 170                 175
   ctg tta tac aaa aca gaa caa gct ggc aac aag gag caa tat tac tcc        576
   Leu Leu Tyr Lys Thr Glu Gln Ala Gly Asn Lys Glu Gln Tyr Tyr Ser
           180                 185                 190
   gaa atc att gat cag atc att gaa ctt ggc caa gcc gat aag ctg atc        624
   Glu Ile Ile Asp Gln Ile Ile Glu Leu Gly Gln Ala Asp Lys Leu Ile
       195                 200                 205
   acc ggc ctt gct tac agc gtt cag cga ttg gtg gtc gac cat ctg cat        672
   Thr Gly Leu Ala Tyr Ser Val Gln Arg Leu Val Val Asp His Leu His
       210                 215                 220
   gtg gtc ggc gat att tat gac cgc ggc ccg cag ccg gat aga att atg        720
   Val Val Gly Asp Ile Tyr Asp Arg Gly Pro Gln Pro Asp Arg Ile Met
   225                 230                 235                 240
   gaa gaa ctg atc aac tat cat tct gtc gat att cag tgg gga aat cac        768
   Glu Glu Leu Ile Asn Tyr His Ser Val Asp Ile Gln Trp Gly Asn His
                   245                 250                 255
   gat gtc ctt tgg atc ggc gcc tat tcc ggt tcc aaa gtg tgc ctg gcc        816
   Asp Val Leu Trp Ile Gly Ala Tyr Ser Gly Ser Lys Val Cys Leu Ala
               260                 265                 270
   aat att atc cgc atc tgt gcc cgc tac gac aac ctg gat att att gag        864
   Asn Ile Ile Arg Ile Cys Ala Arg Tyr Asp Asn Leu Asp Ile Ile Glu
           275                 280                 285
   gac gtg tac ggc atc aac ctg aga ccg ctg ctg aac ctg gcc gaa aaa        912
   Asp Val Tyr Gly Ile Asn Leu Arg Pro Leu Leu Asn Leu Ala Glu Lys
       290                 295                 300
   tat tat gat gat aat cca gcg ttc cgt cca aaa gca gac gaa aac agg        960
   Tyr Tyr Asp Asp Asn Pro Ala Phe Arg Pro Lys Ala Asp Glu Asn Arg
   305                 310                 315                 320
   cca gag gat gag att aag caa atc aca aaa atc cat caa gcg att gcc       1008
   Pro Glu Asp Glu Ile Lys Gln Ile Thr Lys Ile His Gln Ala Ile Ala
                   325                 330                 335
   atg atc caa ttc aag ctt gag agc ccg att atc aag aga cgg ccg aac       1056
   Met Ile Gln Phe Lys Leu Glu Ser Pro Ile Ile Lys Arg Arg Pro Asn
               340                 345                 350
   ttt aat atg gaa gag cgg ctg tta tta gag aaa ata gac tat gac aaa       1104
   Phe Asn Met Glu Glu Arg Leu Leu Leu Glu Lys Ile Asp Tyr Asp Lys
           355                 360                 365
   aat gaa atc acg ctg aac gga aaa aca tat caa ctg gaa aac acc tgc       1152
   Asn Glu Ile Thr Leu Asn Gly Lys Thr Tyr Gln Leu Glu Asn Thr Cys
       370                 375                 380
   ttt gcg acg att aat ccg gag cag cca gat cag cta tta gaa gaa gaa       1200
   Phe Ala Thr Ile Asn Pro Glu Gln Pro Asp Gln Leu Leu Glu Glu Glu
   385                 390                 395                 400
   gca gaa gtc ata gac aag ctg cta ttc tct gtc cag cat tcc gaa aag       1248
   Ala Glu Val Ile Asp Lys Leu Leu Phe Ser Val Gln His Ser Glu Lys
                   405                 410                 415
   ctg ggc cgc cat atg aat ttt atg atg aaa aaa ggc agc ctt tat tta       1296
   Leu Gly Arg His Met Asn Phe Met Met Lys Lys Gly Ser Leu Tyr Leu
               420                 425                 430
   aaa tat aac ggc aac ctg ttg att cac ggc tgt att cca gtt gat gaa       1344
   Lys Tyr Asn Gly Asn Leu Leu Ile His Gly Cys Ile Pro Val Asp Glu
           435                 440                 445
   aac ggc aat atg gaa acg atg atg att gag gat aaa ccg tat gcg ggc       1392
   Asn Gly Asn Met Glu Thr Met Met Ile Glu Asp Lys Pro Tyr Ala Gly
       450                 455                 460
   cgt gag ctg ctc gat gta ttt gaa cga ttc ttg cgg gaa gcc ttt gcc       1440
   Arg Glu Leu Leu Asp Val Phe Glu Arg Phe Leu Arg Glu Ala Phe Ala
   465                 470                 475                 480
   cac ccg gaa gaa acc gat gac ctg gcg aca gat atg gct tgg tat tta       1488
   His Pro Glu Glu Thr Asp Asp Leu Ala Thr Asp Met Ala Trp Tyr Leu
                       485                 490                 495
   tgg aca ggc gaa tac tcc tcc ctc ttc gga aaa cgc gcc atg acg aca      1536
   Trp Thr Gly Glu Tyr Ser Ser Leu Phe Gly Lys Arg Ala Met Thr Thr
                   500                 505                 510
   ttt gag cgc tat ttc atc aaa gag aag gaa acg cat aaa gag aag aaa      1584
   Phe Glu Arg Tyr Phe Ile Lys Glu Lys Glu Thr His Lys Glu Lys Lys
               515                 520                 525
   aac ccg tat tat tat tta cga gaa gac gag gca acc tgc cga aac atc      1632
   Asn Pro Tyr Tyr Tyr Leu Arg Glu Asp Glu Ala Thr Cys Arg Asn Ile
       530                 535                 540
   ctg gca gaa ttc ggc ctc aat cca gat cac ggc cat atc atc aac ggc      1680
   Leu Ala Glu Phe Gly Leu Asn Pro Asp His Gly His Ile Ile Asn Gly
   545                 550                 555                 560
   cat aca cct gta aaa gaa atc gaa gga gaa gac cca atc aaa gca aac      1728
   His Thr Pro Val Lys Glu Ile Glu Gly Glu Asp Pro Ile Lys Ala Asn
                   565                 570                 575
   gga aaa atg atc gtc atc gac ggc ggc ttc tcc aaa gcc tac caa tcc      1776
   Gly Lys Met Ile Val Ile Asp Gly Gly Phe Ser Lys Ala Tyr Gln Ser
                   580                 585                 590
   aca aca ggc atc gcc ggc tac acg ctg cta tac aac tcc tac ggc atg      1824
   Thr Thr Gly Ile Ala Gly Tyr Thr Leu Leu Tyr Asn Ser Tyr Gly Met
               595                 600                 605
   cag ctc gtc gcc cat aaa cac ttc aat tcc aag gca gaa gtc cta agc      1872
   Gln Leu Val Ala His Lys His Phe Asn Ser Lys Ala Glu Val Leu Ser
       610                 615                 620
   acc gga acc gac gtc tta acg gtc aaa cga tta gtg gac aaa gag ctt      1920
   Thr Gly Thr Asp Val Leu Thr Val Lys Arg Leu Val Asp Lys Glu Leu
   625                 630                 635                 640
   gag cgg aag aaa gtg aag gaa acg aat gtg ggt gag gaa ttg ttg cag      1968
   Glu Arg Lys Lys Val Lys Glu Thr Asn Val Gly Glu Glu Leu Leu Gln
                   645                 650                 655
   gaa gtt gcg att tta gag agt ttg cgg gag tat cgg tat atg aag          2013
   Glu Val Ala Ile Leu Glu Ser Leu Arg Glu Tyr Arg Tyr Met Lys
                   660                 665                 670
<210> 2
   <211> 671
   <212> PRT
   <213> Bacillus subtilis
<400> 2
   Met Phe Lys Asn Asn Val Ile Leu Leu Asn Ser Pro Tyr His Ala His
   1               5                   10                  15
   Ala His Lys Glu Gly Phe Ile Leu Lys Arg Gly Trp Thr Val Leu Glu
               20                  25                  30
   Ser Lys Tyr Leu Asp Leu Leu Ala Gln Lys Tyr Asp Cys Glu Glu Lys
           35                  40                  45
   Val Val Thr Glu Ile Ile Asn Leu Lys Ala Ile Leu Asn Leu Pro Lys
       50                  55                  60
   Gly Thr Glu His Phe Val Ser Asp Leu His Gly Glu Tyr Gln Ala Phe
   65                  70                  75                  80
   Gln His Val Leu Arg Asn Gly Ser Gly Arg Val Lys Glu Lys Ile Arg
                   85                  90                  95
   Asp Ile Phe Ser Gly Val Ile Tyr Asp Arg Glu Ile Asp Glu Leu Ala
               100                 105                 110
   Ala Leu Val Tyr Tyr Pro Glu Asp Lys Leu Lys Leu Ile Lys His Asp
           115                 120                 125
   Phe Asp Ala Lys Glu Ala Leu Asn Glu Trp Tyr Lys Glu Thr Ile His
       130                 135                 140
   Arg Met Ile Lys Leu Val Ser Tyr Cys Ser Ser Lys Tyr Thr Arg Ser
   145                 150                 155                 160
   Lys Leu Arg Lys Ala Leu Pro Ala Gln Phe Ala Tyr Ile Thr Glu Glu
                   165                 170                 175
   Leu Leu Tyr Lys Thr Glu Gln Ala Gly Asn Lys Glu Gln Tyr Tyr Ser
               180                 185                 190
   Glu Ile Ile Asp Gln Ile Ile Glu Leu Gly Gln Ala Asp Lys Leu Ile
           195                 200                 205
   Thr Gly Leu Ala Tyr Ser Val Gln Arg Leu Val Val Asp His Leu His
       210                 215                 220
   Val Val Gly Asp Ile Tyr Asp Arg Gly Pro Gln Pro Asp Arg Ile Met
   225                 230                 235                 240
   Glu Glu Leu Ile Asn Tyr His Ser Val Asp Ile Gln Trp Gly Asn His
                   245                 250                 255
   Asp Val Leu Trp Ile Gly Ala Tyr Ser Gly Ser Lys Val Cys Leu Ala
               260                 265                 270
   Asn Ile Ile Arg Ile Cys Ala Arg Tyr Asp Asn Leu Asp Ile Ile Glu
           275                 280                 285
   Asp Val Tyr Gly Ile Asn Leu Arg Pro Leu Leu Asn Leu Ala Glu Lys
       290                 295                 300
   Tyr Tyr Asp Asp Asn Pro Ala Phe Arg Pro Lys Ala Asp Glu Asn Arg
   305                 310                 315                 320
   Pro Glu Asp Glu Ile Lys Gln Ile Thr Lys Ile His Gln Ala Ile Ala
                   325                 330                 335
   Met Ile Gln Phe Lys Leu Glu Ser Pro Ile Ile Lys Arg Arg Pro Asn
               340                 345                 350
   Phe Asn Met Glu Glu Arg Leu Leu Leu Glu Lys Ile Asp Tyr Asp Lys
           355                 360                 365
   Asn Glu Ile Thr Leu Asn Gly Lys Thr Tyr Gln Leu Glu Asn Thr Cys
       370                 375                 380
   Phe Ala Thr Ile Asn Pro Glu Gln Pro Asp Gln Leu Leu Glu Glu Glu
   385                 390                 395                 400
   Ala Glu Val Ile Asp Lys Leu Leu Phe Ser Val Gln His Ser Glu Lys
                   405                 410                 415
   Leu Gly Arg His Met Asn Phe Met Met Lys Lys Gly Ser Leu Tyr Leu
               420                 425                 430
   Lys Tyr Asn Gly Asn Leu Leu Ile His Gly Cys Ile Pro Val Asp Glu
           435                 440                 445
   Asn Gly Asn Met Glu Thr Met Met Ile Glu Asp Lys Pro Tyr Ala Gly
       450                 455                 460
   Arg Glu Leu Leu Asp Val Phe Glu Arg Phe Leu Arg Glu Ala Phe Ala
   465                 470                 475                 480
   His Pro Glu Glu Thr Asp Asp Leu Ala Thr Asp Met Ala Trp Tyr Leu
                   485                 490                 495
   Trp Thr Gly Glu Tyr Ser Ser Leu Phe Gly Lys Arg Ala Met Thr Thr
               500                 505                 510
   Phe Glu Arg Tyr Phe Ile Lys Glu Lys Glu Thr His Lys Glu Lys Lys
           515                 520                 525
   Asn Pro Tyr Tyr Tyr Leu Arg Glu Asp Glu Ala Thr Cys Arg Asn Ile
       530                 535                 540
   Leu Ala Glu Phe Gly Leu Asn Pro Asp His Gly His Ile Ile Asn Gly
   545                 550                 555                 560
   His Thr Pro Val Lys Glu Ile Glu Gly Glu Asp Pro Ile Lys Ala Asn
                   565                 570                 575
   Gly Lys Met Ile Val Ile Asp Gly Gly Phe Ser Lys Ala Tyr Gln Ser
               580                 585                 590
   Thr Thr Gly Ile Ala Gly Tyr Thr Leu Leu Tyr Asn Ser Tyr Gly Met
           595                 600                 605
   Gln Leu Val Ala His Lys His Phe Asn Ser Lys Ala Glu Val Leu Ser
       610                 615                 620
   Thr Gly Thr Asp Val Leu Thr Val Lys Arg Leu Val Asp Lys Glu Leu
   625                 630                 635                 640
   Glu Arg Lys Lys Val Lys Glu Thr Asn Val Gly Glu Glu Leu Leu Gln
                   645                 650                 655
   Glu Val Ala Ile Leu Glu Ser Leu Arg Glu Tyr Arg Tyr Met Lys
               660                 665                 670
<210> 3
   <211> 954
   <212> DNA
   <213> Bacillus subtilis
<220>
   <221> CDS
   <222> (1)..(954)
<400> 3
   atg tct aat caa tac gga gat aag aat tta aag att ttt tct ttg aat       48
   Met Ser Asn Gln Tyr Gly Asp Lys Asn Leu Lys Ile Phe Ser Leu Asn
   1               5                   10                  15
   tcg aat cca gag ctt gca aaa gaa atc gca gat ata gtt gga gtt caa       96
   Ser Asn Pro Glu Leu Ala Lys Glu Ile Ala Asp Ile Val Gly Val Gln
               20                  25                  30
   tta ggg aaa tgt tct gtc aca aga ttt agt gac ggg gaa gtc caa att      144
   Leu Gly Lys Cys Ser Val Thr Arg Phe Ser Asp Gly Glu Val Gln Ile
           35                  40                  45
   aat atc gaa gaa agt att cgc gga tgt gat tgt tac atc atc cag tct      192
   Asn Ile Glu Glu Ser Ile Arg Gly Cys Asp Cys Tyr Ile Ile Gln Ser
       50                  55                  60
   aca agt gac ccc gtt aac gag cat att atg gaa ctg ctg att atg gta      240
   Thr Ser Asp Pro Val Asn Glu His Ile Met Glu Leu Leu Ile Met Val
   65                  70                  75                  80
   gat gcg tta aaa cgc gct tct gca aaa acg att aac att gtt att cct      288
   Asp Ala Leu Lys Arg Ala Ser Ala Lys Thr Ile Asn Ile Val Ile Pro
                   85                  90                  95
   tat tac ggt tat gcg cgt caa gac aga aaa gca aga tcc cgt gag cca      336
   Tyr Tyr Gly Tyr Ala Arg Gln Asp Arg Lys Ala Arg Ser Arg Glu Pro
               100                 105                 110
   atc aca gct aaa ctt ttc gct aac ctg ctt gaa aca gcc ggt gcg act      384
   Ile Thr Ala Lys Leu Phe Ala Asn Leu Leu Glu Thr Ala Gly Ala Thr
           115                 120                 125
   cgt gtg att gca ctt gac ctg cat gcg ccg caa att caa gga ttc ttt      432
   Arg Val Ile Ala Leu Asp Leu His Ala Pro Gln Ile Gln Gly Phe Phe
       130                 135                 140
   gat ata ccg att gac cac tta atg ggt gtt ccg att tta gga gaa tat      480
   Asp Ile Pro Ile Asp His Leu Met Gly Val Pro Ile Leu Gly Glu Tyr
   145                 150                 155                 160
   ttt gaa ggc aaa aat ctt gaa gat atc gtc att gtt tca cca gac cat      528
   Phe Glu Gly Lys Asn Leu Glu Asp Ile Val Ile Val Ser Pro Asp His
                   165                 170                 175
   ggc ggt gtg aca cgt gcc cgc aaa ctg gct gac cga cta aaa gcg cca      576
   Gly Gly Val Thr Arg Ala Arg Lys Leu Ala Asp Arg Leu Lys Ala Pro
               180                 185                 190
   att gcg att atc gat aaa cgc cgt ccg cgt cca aac gtg gcg gaa gtc      624
   Ile Ala Ile Ile Asp Lys Arg Arg Pro Arg Pro Asn Val Ala Glu Val
           195                 200                 205
   atg aat att gta ggt aac atc gaa ggg aag act gct atc ctc atc gat      672
   Met Asn Ile Val Gly Asn Ile Glu Gly Lys Thr Ala Ile Leu Ile Asp
       210                     215                     220
   gac att att gat act gca ggt acg att aca ctt gct gct aat gcg ctc       720
   Asp Ile Ile Asp Thr Ala Gly Thr Ile Thr Leu Ala Ala Asn Ala Leu
   225                     230                     235                 240
   gtt gaa aac gga gcg aaa gaa gta tat gca tgc tgt aca cac cct gta       768
   Val Glu Asn Gly Ala Lys Glu Val Tyr Ala Cys Cys Thr His Pro Val
                   245                     250                     255
   cta tca ggc cct gcg gtt gaa cgg att aat aat tca aca att aaa gag       816
   Leu Ser Gly Pro Ala Val Glu Arg Ile Asn Asn Ser Thr Ile Lys Glu
               260                     265                     270
   ctt gtt gtg aca aac agc atc aag ctt cct gaa gaa aag aaa att gaa       864
   Leu Val Val Thr Asn Ser Ile Lys Leu Pro Glu Glu Lys Lys Ile Glu
           275                     280                     285
   cgc ttt aag cag ctt tca gtc gga ccg ctt ctg gcc gaa gcg att att       912
   Arg Phe Lys Gln Leu Ser Val Gly Pro Leu Leu Ala Glu Ala Ile Ile
       290                     295                     300
   cgc gtt cat gag cag caa tca gtc agc tat ctg ttc agc taa               954
   Arg Val His Glu Gln Gln Ser Val Ser Tyr Leu Phe Ser
   305                     310                     315
<210> 4
   <211> 317
   <212> PRT
   <213> Bacillus subtilis
<400> 4
   Met Ser Asn Gln Tyr Gly Asp Lys Asn Leu Lys Ile Phe Ser Leu Asn
   1               5                   10                  15
   Ser Asn Pro Glu Leu Ala Lys Glu Ile Ala Asp Ile Val Gly Val Gln
               20                  25                  30
   Leu Gly Lys Cys Ser Val Thr Arg Phe Ser Asp Gly Glu Val Gln Ile
           35                  40                  45
   Asn Ile Glu Glu Ser Ile Arg Gly Cys Asp Cys Tyr Ile Ile Gln Ser
       50                  55                  60
   Thr Ser Asp Pro Val Asn Glu His Ile Met Glu Leu Leu Ile Met Val
   65                  70                  75                  80
   Asp Ala Leu Lys Arg Ala Ser Ala Lys Thr Ile Asn Ile Val Ile Pro
                   85                  90                  95
   Tyr Tyr Gly Tyr Ala Arg Gln Asp Arg Lys Ala Arg Ser Arg Glu Pro
               100                 105                 110
   Ile Thr Ala Lys Leu Phe Ala Asn Leu Leu Glu Thr Ala Gly Ala Thr
           115                 120                 125
   Arg Val Ile Ala Leu Asp Leu His Ala Pro Gln Ile Gln Gly Phe Phe
       130                 135                 140
   Asp Ile Pro Ile Asp His Leu Met Gly Val Pro Ile Leu Gly Glu Tyr
   145                 150                 155                 160
   Phe Glu Gly Lys Asn Leu Glu Asp Ile Val Ile Val Ser Pro Asp His
                   165                 170                 175
   Gly Gly Val Thr Arg Ala Arg Lys Leu Ala Asp Arg Leu Lys Ala Pro
               180                 185                 190
   Ile Ala Ile Ile Asp Lys Arg Arg Pro Arg Pro Asn Val Ala Glu Val
           195                 200                 205
   Met Asn Ile Val Gly Asn Ile Glu Gly Lys Thr Ala Ile Leu Ile Asp
       210                 215                 220
   Asp Ile Ile Asp Thr Ala Gly Thr Ile Thr Leu Ala Ala Asn Ala Leu
   225                 230                 235                 240
   Val Glu Asn Gly Ala Lys Glu Val Tyr Ala Cys Cys Thr His Pro Val
                   245                 250                 255
   Leu Ser Gly Pro Ala Val Glu Arg Ile Asn Asn Ser Thr Ile Lys Glu
               260                 265                 270
   Leu Val Val Thr Asn Ser Ile Lys Leu Pro Glu Glu Lys Lys Ile Glu
           275                 280                 285
   Arg Phe Lys Gln Leu Ser Val Gly Pro Leu Leu Ala Glu Ala Ile Ile
       290                 295                 300
   Arg Val His Glu Gln Gln Ser Val Ser Tyr Leu Phe Ser
   305                 310                 315
<210> 5
   <211> 858
   <212> DNA
   <213> Bacillus subtilis
<220>
   <221> CDS
   <222> (1)..(858)
<400> 5
   atg aag ttt cgt cgc agc ggc aga ttg gtg gac tta aca aat tat ttg      48
   Met Lys Phe Arg Arg Ser Gly Arg Leu Val Asp Leu Thr Asn Tyr Leu
   1               5                   10                  15
   tta acc cat ccg cac gag tta ata ccg cta acc ttt ttc tct gag cgg      96
   Leu Thr His Pro His Glu Leu Ile Pro Leu Thr Phe Phe Ser Glu Arg
               20                  25                  30
   tat gaa tct gca aaa tca tcg atc agt gaa gat tta aca att att aaa      144
   Tyr Glu Ser Ala Lys Ser Ser Ile Ser Glu Asp Leu Thr Ile Ile Lys
           35                  40                  45
   caa acc ttt gaa cag cag ggg att ggt act ttg ctt act gtt ccc gga      192
   Gln Thr Phe Glu Gln Gln Gly Ile Gly Thr Leu Leu Thr Val Pro Gly
       50                  55                  60
   gct gcc gga ggc gtt aaa tat att ccg aaa atg aag cag gct gaa gct      240
   Ala Ala Gly Gly Val Lys Tyr Ile Pro Lys Met Lys Gln Ala Glu Ala
   65                  70                  75                  80
   gaa gag ttt gtg cag aca ctt gga cag tcg ctg gca aat cct gag cgt      288
   Glu Glu Phe Val Gln Thr Leu Gly Gln Ser Leu Ala Asn Pro Glu Arg
                   85                  90                  95
   atc ctt ccg ggc ggt tat gta tat tta acg gat atc tta gga aag cca      336
   Ile Leu Pro Gly Gly Tyr Val Tyr Leu Thr Asp Ile Leu Gly Lys Pro
               100                 105                 110
   tct gta ctc tcc aag gta ggg aag ctg ttt gct tcc gtg ttt gca gag      384
   Ser Val Leu Ser Lys Val Gly Lys Leu Phe Ala Ser Val Phe Ala Glu
           115                 120                 125
   cgc gaa att gat gtt gtc atg acc gtt gcc acg aaa ggc atc cct ctt      432
   Arg Glu Ile Asp Val Val Met Thr Val Ala Thr Lys Gly Ile Pro Leu
       130                 135                 140
   gcg tac gca gct gca agc tat ttg aat gtg cct gtt gtg atc gtt cgt      480
   Ala Tyr Ala Ala Ala Ser Tyr Leu Asn Val Pro Val Val Ile Val Arg
   145                 150                 155                 160
   aaa gac aat aag gta aca gag ggc tcc aca gtc agc att aat tac gtt      528
   Lys Asp Asn Lys Val Thr Glu Gly Ser Thr Val Ser Ile Asn Tyr Val
                   165                 170                 175
   tca ggc tcc tca aac cgc att caa aca atg tca ctt gcg aaa aga agc      576
   Ser Gly Ser Ser Asn Arg Ile Gln Thr Met Ser Leu Ala Lys Arg Ser
               180                 185                 190
   atg aaa acg ggt tca aac gta ctc att att gat gac ttt atg aaa gca      624
   Met Lys Thr Gly Ser Asn Val Leu Ile Ile Asp Asp Phe Met Lys Ala
           195                 200                 205
   ggc ggc acc att aat ggt atg att aac ctg ttg gat gag ttt aac gca      672
   Gly Gly Thr Ile Asn Gly Met Ile Asn Leu Leu Asp Glu Phe Asn Ala
       210                 215                 220
   aat gtg gcg gga atc ggc gtc tta gtt gaa gcc gaa gga gta gat gaa      720
   Asn Val Ala Gly Ile Gly Val Leu Val Glu Ala Glu Gly Val Asp Glu
   225                 230                 235                 240
   cgt ctt gtt gac gaa tat atg tca ctt ctt act ctt tca acc atc aac      768
   Arg Leu Val Asp Glu Tyr Met Ser Leu Leu Thr Leu Ser Thr Ile Asn
                   245                 250                 255
   atg aaa gag aag tcc att gaa att cag aat ggc aat ttt ctg cgt ttt      816
   Met Lys Glu Lys Ser Ile Glu Ile Gln Asn Gly Asn Phe Leu Arg Phe
               260                 265                 270
   ttt aaa gac aat ctt tta aag aat gga gag aca gaa tca tga              858
   Phe Lys Asp Asn Leu Leu Lys Asn Gly Glu Thr Glu Ser
           275                 280                 285
<210> 6
   <211> 285
   <212> PRT
   <213> Bacillus subtilis
<400> 6
   Met Lys Phe Arg Arg Ser Gly Arg Leu Val Asp Leu Thr Asn Tyr Leu
   1               5                   10                  15
   Leu Thr His Pro His Glu Leu Ile Pro Leu Thr Phe Phe Ser Glu Arg
               20                  25                  30
   Tyr Glu Ser Ala Lys Ser Ser Ile Ser Glu Asp Leu Thr Ile Ile Lys
           35                  40                  45
   Gln Thr Phe Glu Gln Gln Gly Ile Gly Thr Leu Leu Thr Val Pro Gly
       50                  55                  60
   Ala Ala Gly Gly Val Lys Tyr Ile Pro Lys Met Lys Gln Ala Glu Ala
   65                  70                  75                  80
   Glu Glu Phe Val Gln Thr Leu Gly Gln Ser Leu Ala Asn Pro Glu Arg
                   85                  90                  95
   Ile Leu Pro Gly Gly Tyr Val Tyr Leu Thr Asp Ile Leu Gly Lys Pro
               100                 105                 110
   Ser Val Leu Ser Lys Val Gly Lys Leu Phe Ala Ser Val Phe Ala Glu
           115                 120                 125
   Arg Glu Ile Asp Val Val Met Thr Val Ala Thr Lys Gly Ile Pro Leu
       130                 135                 140
   Ala Tyr Ala Ala Ala Ser Tyr Leu Asn Val Pro Val Val Ile Val Arg
   145                 150                 155                 160
   Lys Asp Asn Lys Val Thr Glu Gly Ser Thr Val Ser Ile Asn Tyr Val
                   165                 170                 175
   Ser Gly Ser Ser Asn Arg Ile Gln Thr Met Ser Leu Ala Lys Arg Ser
               180                 185                 190
   Met Lys Thr Gly Ser Asn Val Leu Ile Ile Asp Asp Phe Met Lys Ala
           195                 200                 205
   Gly Gly Thr Ile Asn Gly Met Ile Asn Leu Leu Asp Glu Phe Asn Ala
       210                 215                 220
   Asn Val Ala Gly Ile Gly Val Leu Val Glu Ala Glu Gly Val Asp Glu
   225                 230                 235                 240
   Arg Leu Val Asp Glu Tyr Met Ser Leu Leu Thr Leu Ser Thr Ile Asn
                   245                 250                 255
   Met Lys Glu Lys Ser Ile Glu Ile Gln Asn Gly Asn Phe Leu Arg Phe
               260                 265                 270
   Phe Lys Asp Asn Leu Leu Lys Asn Gly Glu Thr Glu Ser
           275                 280                 285
<210> 7
   <211> 702
   <212> DNA
   <213> Bacillus subtilis
<220>
   <221> CDS
   <222> (1)..(702)
<400> 7
   atg agt gta cat ata ggt gct gaa aaa gga caa att gcg gat act gtg        48
   Met Ser Val His Ile Gly Ala Glu Lys Gly Gln Ile Ala Asp Thr Val
   1               5                   10                  15
   ctt ttg ccg gga gat cct ctc aga gca aaa ttt att gca gaa acg tat        96
   Leu Leu Pro Gly Asp Pro Leu Arg Ala Lys Phe Ile Ala Glu Thr Tyr
               20                  25                  30
   ctt gaa aat gta gaa tgc tac aat gaa gtc aga ggc atg tat gga ttt        144
   Leu Glu Asn Val Glu Cys Tyr Asn Glu Val Arg Gly Met Tyr Gly Phe
                   35                      40                      45
   acg ggt aca tat aaa ggt aaa aaa atc tca gta caa ggc acg gga atg       192
   Thr Gly Thr Tyr Lys Gly Lys Lys Ile Ser Val Gln Gly Thr Gly Met
           50                      55                      60
   gga gtt ccg tct att tca att tat gtg aat gaa tta att caa agc tac       240
   Gly Val Pro Ser Ile Ser Ile Tyr Val Asn Glu Leu Ile Gln Ser Tyr
   65                      70                      75                  80
   gat gtg caa aat cta ata aga gtc ggt tcc tgc ggc gct att cgt aaa       288
   Asp Val Gln Asn Leu Ile Arg Val Gly Ser Cys Gly Ala Ile Arg Lys
                       85                      90                  95
   gat gtc aaa gtg cga gac gtc att ttg gcg atg acc tcc tca act gat       336
   Asp Val Lys Val Arg Asp Val Ile Leu Ala Met Thr Ser Ser Thr Asp
               100                     105                     110
   tca caa atg aac aga gtt gct ttc gga agc gtt gat ttt gcg cct tgc       384
   Ser Gln Met Asn Arg Val Ala Phe Gly Ser Val Asp Phe Ala Pro Cys
           115                     120                     125
   gca gat ttc gag ctt tta aaa aat gcc tat gat gcc gca aag gat aaa       432
   Ala Asp Phe Glu Leu Leu Lys Asn Ala Tyr Asp Ala Ala Lys Asp Lys
       130                     135                     140
   ggt gtg ccg gtg act gta gga agc gta ttt aca gct gac cag ttc tac       480
   Gly Val Pro Val Thr Val Gly Ser Val Phe Thr Ala Asp Gln Phe Tyr
   145                     150                     155                 160
   aat gac gat tcg caa att gaa aaa ctt gca aaa tac ggt gtg ctt ggc       528
   Asn Asp Asp Ser Gln Ile Glu Lys Leu Ala Lys Tyr Gly Val Leu Gly
                   165                     170                     175
   gtt gaa atg gaa aca act gca ttg tat aca tta gca gcg aag cac gga       576
   Val Glu Met Glu Thr Thr Ala Leu Tyr Thr Leu Ala Ala Lys His Gly
               180                     185                     190
   aga aaa gcc ctg tca att ctc acc gtg agt gat cac gta tta aca gga       624
   Arg Lys Ala Leu Ser Ile Leu Thr Val Ser Asp His Val Leu Thr Gly
           195                     200                     205
   gaa gaa acg aca gcg gaa gag cgt caa acg aca ttt cat gat atg ata       672
   Glu Glu Thr Thr Ala Glu Glu Arg Gln Thr Thr Phe His Asp Met Ile
       210                     215                     220
   gaa gtg gct tta cat tcc gta tca caa taa                               702
   Glu Val Ala Leu His Ser Val Ser Gln
   225                     230
<210> 8
   <211> 233
   <212> PRT
   <213> Bacillus subtilis
<400> 8
   Met Ser Val His Ile Gly Ala Glu Lys Gly Gln Ile Ala Asp Thr Val
   1               5                   10                  15
   Leu Leu Pro Gly Asp Pro Leu Arg Ala Lys Phe Ile Ala Glu Thr Tyr
               20                  25                  30
   Leu Glu Asn Val Glu Cys Tyr Asn Glu Val Arg Gly Met Tyr Gly Phe
           35                  40                  45
   Thr Gly Thr Tyr Lys Gly Lys Lys Ile Ser Val Gln Gly Thr Gly Met
       50                  55                  60
   Gly Val Pro Ser Ile Ser Ile Tyr Val Asn Glu Leu Ile Gln Ser Tyr
   65                  70                  75                  80
   Asp Val Gln Asn Leu Ile Arg Val Gly Ser Cys Gly Ala Ile Arg Lys
                   85                  90                  95
   Asp Val Lys Val Arg Asp Val Ile Leu Ala Met Thr Ser Ser Thr Asp
               100                 105                 110
   Ser Gln Met Asn Arg Val Ala Phe Gly Ser Val Asp Phe Ala Pro Cys
           115                 120                 125
   Ala Asp Phe Glu Leu Leu Lys Asn Ala Tyr Asp Ala Ala Lys Asp Lys
       130                 135                 140
   Gly Val Pro Val Thr Val Gly Ser Val Phe Thr Ala Asp Gln Phe Tyr
   145                 150                 155                 160
   Asn Asp Asp Ser Gln Ile Glu Lys Leu Ala Lys Tyr Gly Val Leu Gly
                   165                 170                 175
   Val Glu Met Glu Thr Thr Ala Leu Tyr Thr Leu Ala Ala Lys His Gly
               180                 185                 190
   Arg Lys Ala Leu Ser Ile Leu Thr Val Ser Asp His Val Leu Thr Gly
           195                 200                 205
   Glu Glu Thr Thr Ala Glu Glu Arg Gln Thr Thr Phe His Asp Met Ile
       210                 215                 220
   Glu Val Ala Leu His Ser Val Ser Gln
   225                 230
<210> 9
   <211> 816
   <212> DNA
   <213> Bacillus subtilis
<220>
   <221> CDS
   <222> (1)..(816)
<400> 9
   ttg aag gac aga att gaa cgc gca gcc gct ttt att aaa caa aac ctg      48
   Leu Lys Asp Arg Ile Glu Arg Ala Ala Ala Phe Ile Lys Gln Asn Leu
   1               5                   10                  15
   ccg gaa tct cca aag atc ggc ctt att tta ggc tca ggt ctt ggc att      96
   Pro Glu Ser Pro Lys Ile Gly Leu Ile Leu Gly Ser Gly Leu Gly Ile
               20                  25                  30
   ttg gcg gac gaa atc gaa aat ccg gtc aag ctg aaa tat gaa gat ata     144
   Leu Ala Asp Glu Ile Glu Asn Pro Val Lys Leu Lys Tyr Glu Asp Ile
           35                  40                  45
   cct gaa ttc ccg gta tct act gtt gaa ggg cat gcc gga cag ctt gtg     192
   Pro Glu Phe Pro Val Ser Thr Val Glu Gly His Ala Gly Gln Leu Val
       50                  55                  60
   ctt ggc act ctt gaa gga gtt tcc gtc att gca atg cag ggc cgc ttt     240
   Leu Gly Thr Leu Glu Gly Val Ser Val Ile Ala Met Gln Gly Arg Phe
   65                  70                  75                  80
   cat ttt tat gaa ggc tac tca atg gag aaa gtc aca ttc cct gta cgc     288
   His Phe Tyr Glu Gly Tyr Ser Met Glu Lys Val Thr Phe Pro Val Arg
                       85                      90                      95
   gtg atg aaa gcg ctc ggt gtg gaa gcg ttg atc gtg aca aat gcc gca      336
   Val Met Lys Ala Leu Gly Val Glu Ala Leu Ile Val Thr Asn Ala Ala
                   100                     105                     110
   ggc ggt gtc aac act gaa ttc cgt gcg gga gat tta atg att att acc      384
   Gly Gly Val Asn Thr Glu Phe Arg Ala Gly Asp Leu Met Ile Ile Thr
               115                     120                     125
   gat cat atc aac ttt atg gga aca aac ccg tta atc ggg cca aac gaa      432
   Asp His Ile Asn Phe Met Gly Thr Asn Pro Leu Ile Gly Pro Asn Glu
       130                     135                     140
   gca gat ttc ggc gcc aga ttt cca gat atg tct tca gcc tat gac aaa      480
   Ala Asp Phe Gly Ala Arg Phe Pro Asp Met Ser Ser Ala Tyr Asp Lys
   145                     150                     155                     160
   gat ctg tcc agc ctg gct gaa aag att gcg aaa gac ctt aat atc cca      528
   Asp Leu Ser Ser Leu Ala Glu Lys Ile Ala Lys Asp Leu Asn Ile Pro
                       165                     170                     175
   att caa aaa ggc gtg tac act gct gtg aca gga cct tct tac gaa aca      576
   Ile Gln Lys Gly Val Tyr Thr Ala Val Thr Gly Pro Ser Tyr Glu Thr
                   180                     185                     190
   ccg gca gaa gtc cgt ttc tta aga acg atg ggc tct gat gca gtc ggc      624
   Pro Ala Glu Val Arg Phe Leu Arg Thr Met Gly Ser Asp Ala Val Gly
               195                     200                     205
   atg tct act gtt ccg gaa gtc att gta gcg aat cat gcg gga atg cgg      672
   Met Ser Thr Val Pro Glu Val Ile Val Ala Asn His Ala Gly Met Arg
       210                     215                     220
   gtt ctt ggc att tcc tgc atc tct aac gcg gca gcc gga att ctg gat      720
   Val Leu Gly Ile Ser Cys Ile Ser Asn Ala Ala Ala Gly Ile Leu Asp
   225                     230                     235                     240
   cag cct tta agt cac gat gaa gtt atg gaa gtg acc gaa aaa gta aaa      768
   Gln Pro Leu Ser His Asp Glu Val Met Glu Val Thr Glu Lys Val Lys
                       245                     250                     255
   gct gga ttc tta aag ctt gtt aaa gcg atc gtc gct cag tac gaa taa      816
   Ala Gly Phe Leu Lys Leu Val Lys Ala Ile Val Ala Gln Tyr Glu
                   260                     265                     270
<210> 10
   <211> 271
   <212> PRT
   <213> Bacillus subtilis
<400> 10
   Leu Lys Asp Arg Ile Glu Arg Ala Ala Ala Phe Ile Lys Gln Asn Leu
   1               5                   10                  15
   Pro Glu Ser Pro Lys Ile Gly Leu Ile Leu Gly Ser Gly Leu Gly Ile
               20                  25                  30
   Leu Ala Asp Glu Ile Glu Asn Pro Val Lys Leu Lys Tyr Glu Asp Ile
           35                  40                  45
   Pro Glu Phe Pro Val Ser Thr Val Glu Gly His Ala Gly Gln Leu Val
       50                  55                  60
   Leu Gly Thr Leu Glu Gly Val Ser Val Ile Ala Met Gln Gly Arg Phe
   65                  70                  75                  80
   His Phe Tyr Glu Gly Tyr Ser Met Glu Lys Val Thr Phe Pro Val Arg
                   85                  90                  95
   Val Met Lys Ala Leu Gly Val Glu Ala Leu Ile Val Thr Asn Ala Ala
               100                 105                 110
   Gly Gly Val Asn Thr Glu Phe Arg Ala Gly Asp Leu Met Ile Ile Thr
           115                 120                 125
   Asp His Ile Asn Phe Met Gly Thr Asn Pro Leu Ile Gly Pro Asn Glu
       130                 135                 140
   Ala Asp Phe Gly Ala Arg Phe Pro Asp Met Ser Ser Ala Tyr Asp Lys
   145                 150                 155                 160
   Asp Leu Ser Ser Leu Ala Glu Lys Ile Ala Lys Asp Leu Asn Ile Pro
                   165                 170                 175
   Ile Gln Lys Gly Val Tyr Thr Ala Val Thr Gly Pro Ser Tyr Glu Thr
               180                 185                 190
   Pro Ala Glu Val Arg Phe Leu Arg Thr Met Gly Ser Asp Ala Val Gly
           195                 200                 205
   Met Ser Thr Val Pro Glu Val Ile Val Ala Asn His Ala Gly Met Arg
       210                 215                 220
   Val Leu Gly Ile Ser Cys Ile Ser Asn Ala Ala Ala Gly Ile Leu Asp
   225                 230                 235                 240
   Gln Pro Leu Ser His Asp Glu Val Met Glu Val Thr Glu Lys Val Lys
                   245                 250                 255
   Ala Gly Phe Leu Lys Leu Val Lys Ala Ile Val Ala Gln Tyr Glu
               260                 265                 270
<210> 11
   <211> 1293
   <212> DNA
   <213> Bacillus subtilis
<220>
   <221> CDS
   <222> (1)..(1293)
<400> 11
   atg tct tca gta gtt gta gta ggt acg caa tgg ggc gat gaa gga aaa       48
   Met Ser Ser Val Val Val Val Gly Thr Gln Trp Gly Asp Glu Gly Lys
   1               5                   10                  15
   ggt aaa att aca gat ttc cta tca gaa aat gca gaa gtg atc gcc cgt       96
   Gly Lys Ile Thr Asp Phe Leu Ser Glu Asn Ala Glu Val Ile Ala Arg
               20                  25                  30
   tat caa ggc gga aat aac gca ggg cat aca atc aag ttt gac gga atc      144
   Tyr Gln Gly Gly Asn Asn Ala Gly His Thr Ile Lys Phe Asp Gly Ile
           35                  40                  45
   aca tat aag ctt cac tta atc ccg tct gga att ttc tat aag gat aaa      192
   Thr Tyr Lys Leu His Leu Ile Pro Ser Gly Ile Phe Tyr Lys Asp Lys
       50                  55                  60
   acg tgt gta atc gga aac gga atg gtt gta gat ccg aaa gca tta gtc      240
   Thr Cys Val Ile Gly Asn Gly Met Val Val Asp Pro Lys Ala Leu Val
   65                  70                  75                  80
   aca gag ctt gcg tat ctt cat gag cgc aac gtg agt aca gat aac ctg      288
   Thr Glu Leu Ala Tyr Leu His Glu Arg Asn Val Ser Thr Asp Asn Leu
                   85                  90                  95
   aga atc agc aac aga gct cac gtc att ctg ccg tat cat ttg aaa ttg      336
   Arg Ile Ser Asn Arg Ala His Val Ile Leu Pro Tyr His Leu Lys Leu
               100                 105                 110
   gat gaa gtg gaa gaa gag cgt aaa ggg gct aac aag atc ggc aca acg      384
   Asp Glu Val Glu Glu Glu Arg Lys Gly Ala Asn Lys Ile Gly Thr Thr
           115                 120                 125
   aaa aaa gga atc ggc cct gct tac atg gat aaa gca gcc cgc atc gga      432
   Lys Lys Gly Ile Gly Pro Ala Tyr Met Asp Lys Ala Ala Arg Ile Gly
       130                 135                 140
   att cgc atc gcg gat ctg tta gac cgt gac gcg ttt gcg gaa aag ctt      480
   Ile Arg Ile Ala Asp Leu Leu Asp Arg Asp Ala Phe Ala Glu Lys Leu
   145                 150                 155                 160
   gag cgc aat ctt gaa gaa aaa aac cgt ctt ctc gag aaa atg tac gag      528
   Glu Arg Asn Leu Glu Glu Lys Asn Arg Leu Leu Glu Lys Met Tyr Glu
                           165                 170                 175
   aca gaa ggg ttt aaa ctt gag gat atc tta gac gaa tat tat gag tac       576
   Thr Glu Gly Phe Lys Leu Glu Asp Ile Leu Asp Glu Tyr Tyr Glu Tyr
               180                 185                 190
   gga cag cag att aaa aag tat gtt tgc gat aca tct gtt gtc tta aac       624
   Gly Gln Gln Ile Lys Lys Tyr Val Cys Asp Thr Ser Val Val Leu Asn
           195                 200                 205
   gat gct ctt gat gaa ggg cgc cgt gta tta ttt gaa ggc gca caa ggg       672
   Asp Ala Leu Asp Glu Gly Arg Arg Val Leu Phe Glu Gly Ala Gln Gly
       210                 215                 220
   gtt atg ctc gat atc gac caa gga aca tac ccg ttt gtt acg tca tct       720
   Val Met Leu Asp Ile Asp Gln Gly Thr Tyr Pro Phe Val Thr Ser Ser
   225                 230                 235                 240
   aac ccg gtt gcc ggc ggt gtc acg atc ggt tct ggt gtc ggc ccg acc       768
   Asn Pro Val Ala Gly Gly Val Thr Ile Gly Ser Gly Val Gly Pro Thr
                   245                 250                 255
   aaa atc aag cac gtt gtc ggt gta tca aaa gca tat acg act cgt gtc       816
   Lys Ile Lys His Val Val Gly Val Ser Lys Ala Tyr Thr Thr Arg Val
               260                 265                 270
   ggc gac ggt cct ttt ccg act gag ctg aaa gat gaa atc ggc gat caa       864
   Gly Asp Gly Pro Phe Pro Thr Glu Leu Lys Asp Glu Ile Gly Asp Gln
           275                 280                 285
   atc cgt gaa gtc gga cgc gaa tat gga aca aca aca ggc cgc ccg cgc       912
   Ile Arg Glu Val Gly Arg Glu Tyr Gly Thr Thr Thr Gly Arg Pro Arg
       290                 295                 300
   cgt gtc ggc tgg ttt gac agc gtt gtt gtc cgc cac gcc cgc cgt gtg       960
   Arg Val Gly Trp Phe Asp Ser Val Val Val Arg His Ala Arg Arg Val
   305                 310                 315                 320
   agc gga att aca gat ctt tct ctg aac tca att gac gtc cta gca gga      1008
   Ser Gly Ile Thr Asp Leu Ser Leu Asn Ser Ile Asp Val Leu Ala Gly
                   325                 330                 335
   att gaa acg ttg aaa atc tgt gtg gcg tac cgc tac aaa ggc gaa atc      1056
   Ile Glu Thr Leu Lys Ile Cys Val Ala Tyr Arg Tyr Lys Gly Glu Ile
               340                 345                 350
   att gaa gaa ttc cca gca agt ctt aag gca ctt gct gaa tgt gag ccg      1104
   Ile Glu Glu Phe Pro Ala Ser Leu Lys Ala Leu Ala Glu Cys Glu Pro
           355                 360                 365
   gta tat gaa gaa atg ccg ggc tgg act gag gat att aca ggt gcg aag      1152
   Val Tyr Glu Glu Met Pro Gly Trp Thr Glu Asp Ile Thr Gly Ala Lys
       370                 375                 380
   agc ttg agc gag ctt ccg gaa aat gcg cgc cat tat ctt gag cgt gtg      1200
   Ser Leu Ser Glu Leu Pro Glu Asn Ala Arg His Tyr Leu Glu Arg Val
   385                 390                 395                 400
   tct cag ctg aca ggc att ccg ctt tct att ttc tct gtc ggt cca gac      1248
   Ser Gln Leu Thr Gly Ile Pro Leu Ser Ile Phe Ser Val Gly Pro Asp
                   405                 410                 415
   cgc tca caa aca aat gtc ctt cgc agt gtg tac cgt gcg aac taa          1293
   Arg Ser Gln Thr Asn Val Leu Arg Ser Val Tyr Arg Ala Asn
               420                 425                 430
<210> 12
   <211> 430
   <212> PRT
   <213> Bacillus subtilis
<400> 12
   Met Ser Ser Val Val Val Val Gly Thr Gln Trp Gly Asp Glu Gly Lys
   1               5                   10                  15
   Gly Lys Ile Thr Asp Phe Leu Ser Glu Asn Ala Glu Val Ile Ala Arg
               20                  25                  30
   Tyr Gln Gly Gly Asn Asn Ala Gly His Thr Ile Lys Phe Asp Gly Ile
           35                  40                  45
   Thr Tyr Lys Leu His Leu Ile Pro Ser Gly Ile Phe Tyr Lys Asp Lys
       50                  55                  60
   Thr Cys Val Ile Gly Asn Gly Met Val Val Asp Pro Lys Ala Leu Val
   65                  70                  75                  80
   Thr Glu Leu Ala Tyr Leu His Glu Arg Asn Val Ser Thr Asp Asn Leu
                   85                  90                  95
   Arg Ile Ser Asn Arg Ala His Val Ile Leu Pro Tyr His Leu Lys Leu
               100                 105                 110
   Asp Glu Val Glu Glu Glu Arg Lys Gly Ala Asn Lys Ile Gly Thr Thr
           115                 120                 125
   Lys Lys Gly Ile Gly Pro Ala Tyr Met Asp Lys Ala Ala Arg Ile Gly
       130                 135                 140
   Ile Arg Ile Ala Asp Leu Leu Asp Arg Asp Ala Phe Ala Glu Lys Leu
   145                 150                 155                 160
   Glu Arg Asn Leu Glu Glu Lys Asn Arg Leu Leu Glu Lys Met Tyr Glu
                   165                 170                 175
   Thr Glu Gly Phe Lys Leu Glu Asp Ile Leu Asp Glu Tyr Tyr Glu Tyr
               180                 185                 190
   Gly Gln Gln Ile Lys Lys Tyr Val Cys Asp Thr Ser Val Val Leu Asn
           195                 200                 205
   Asp Ala Leu Asp Glu Gly Arg Arg Val Leu Phe Glu Gly Ala Gln Gly
       210                 215                 220
   Val Met Leu Asp Ile Asp Gln Gly Thr Tyr Pro Phe Val Thr Ser Ser
   225                 230                 235                 240
   Asn Pro Val Ala Gly Gly Val Thr Ile Gly Ser Gly Val Gly Pro Thr
                   245                 250                 255
   Lys Ile Lys His Val Val Gly Val Ser Lys Ala Tyr Thr Thr Arg Val
               260                 265                 270
   Gly Asp Gly Pro Phe Pro Thr Glu Leu Lys Asp Glu Ile Gly Asp Gln
           275                 280                 285
   Ile Arg Glu Val Gly Arg Glu Tyr Gly Thr Thr Thr Gly Arg Pro Arg
       290                 295                 300
   Arg Val Gly Trp Phe Asp Ser Val Val Val Arg His Ala Arg Arg Val
   305                 310                 315                 320
   Ser Gly Ile Thr Asp Leu Ser Leu Asn Ser Ile Asp Val Leu Ala Gly
                   325                 330                 335
   Ile Glu Thr Leu Lys Ile Cys Val Ala Tyr Arg Tyr Lys Gly Glu Ile
               340                 345                 350
   Ile Glu Glu Phe Pro Ala Ser Leu Lys Ala Leu Ala Glu Cys Glu Pro
           355                 360                 365
   Val Tyr Glu Glu Met Pro Gly Trp Thr Glu Asp Ile Thr Gly Ala Lys
       370                 375                 380
   Ser Leu Ser Glu Leu Pro Glu Asn Ala Arg His Tyr Leu Glu Arg Val
   385                 390                 395                 400
   Ser Gln Leu Thr Gly Ile Pro Leu Ser Ile Phe Ser Val Gly Pro Asp
                   405                 410                 415
   Arg Ser Gln Thr Asn Val Leu Arg Ser Val Tyr Arg Ala Asn
               420                 425                 430
<210> 13
   <211> 1542
   <212> DNA
   <213> Bacillus subtilis
<220>
   <221> CDS
   <222> (1)..(1542)
<400> 13
   atg tgg gaa agt aaa ttt tca aaa gaa ggc tta acg ttc gac gat gtg        48
   Met Trp Glu Ser Lys Phe Ser Lys Glu Gly Leu Thr Phe Asp Asp Val
   1               5                   10                  15
   ctg ctt gtg cca gca aag tct gag gta ctt ccg cat gat gtg gat tta        96
   Leu Leu Val Pro Ala Lys Ser Glu Val Leu Pro His Asp Val Asp Leu
               20                  25                  30
   tct gta gaa ctt aca aaa acg tta aag cta aat att cct gtc atc agc       144
   Ser Val Glu Leu Thr Lys Thr Leu Lys Leu Asn Ile Pro Val Ile Ser
           35                  40                  45
   gca ggt atg gac act gta aca gaa tca gca atg gca att gca atg gca       192
   Ala Gly Met Asp Thr Val Thr Glu Ser Ala Met Ala Ile Ala Met Ala
       50                  55                  60
   aga cag ggc ggc ttg ggc atc att cac aaa aat atg tcc att gaa cag       240
   Arg Gln Gly Gly Leu Gly Ile Ile His Lys Asn Met Ser Ile Glu Gln
   65                  70                  75                  80
   cag gct gaa caa gtt gat aaa gta aag cgt tct gag cgc ggc gtt atc       288
   Gln Ala Glu Gln Val Asp Lys Val Lys Arg Ser Glu Arg Gly Val Ile
                   85                  90                  95
   aca aat ccc ttc ttt tta act cct gat cac caa gta ttt gat gcg gag       336
   Thr Asn Pro Phe Phe Leu Thr Pro Asp His Gln Val Phe Asp Ala Glu
               100                 105                 110
   cat ttg atg ggg aaa tac aga att tcc ggt gtt ccg att gta aat aac       384
   His Leu Met Gly Lys Tyr Arg Ile Ser Gly Val Pro Ile Val Asn Asn
           115                 120                 125
   gaa gaa gac cag aag ctt gtt gga att att aca aac cgt gac ctt cgt       432
   Glu Glu Asp Gln Lys Leu Val Gly Ile Ile Thr Asn Arg Asp Leu Arg
       130                 135                 140
   ttt att tct gac tac tca atg aaa atc agc gac gtc atg acg aaa gaa       480
   Phe Ile Ser Asp Tyr Ser Met Lys Ile Ser Asp Val Met Thr Lys Glu
   145                 150                 155                 160
   gag cta gtt act gca tct gta gga act act ctg gat gaa gct gaa aag       528
   Glu Leu Val Thr Ala Ser Val Gly Thr Thr Leu Asp Glu Ala Glu Lys
                   165                 170                 175
   att ttg caa aaa cat aaa att gaa aag ctt cct ctc gta gat gac cag       576
   Ile Leu Gln Lys His Lys Ile Glu Lys Leu Pro Leu Val Asp Asp Gln
               180                 185                 190
   aat aaa tta aaa ggt ctt atc aca att aaa gac att gaa aaa gtc att       624
   Asn Lys Leu Lys Gly Leu Ile Thr Ile Lys Asp Ile Glu Lys Val Ile
           195                 200                 205
   gag ttc ccg aac tca tct aaa gac att cac ggc cgc ctg atc gtt ggc       672
   Glu Phe Pro Asn Ser Ser Lys Asp Ile His Gly Arg Leu Ile Val Gly
       210                 215                 220
   gcg gca gtt ggt gta act ggc gat aca atg act cgc gtc aaa aag ctt       720
   Ala Ala Val Gly Val Thr Gly Asp Thr Met Thr Arg Val Lys Lys Leu
   225                 230                 235                 240
   gtt gaa gcc aat gtt gat gtg att gtt atc gat aca gct cac gga cac       768
   Val Glu Ala Asn Val Asp Val Ile Val Ile Asp Thr Ala His Gly His
                   245                 250                 255
   tct caa ggc gtt tta aac aca gtt aca aaa atc cgt gaa acg tat ccc       816
   Ser Gln Gly Val Leu Asn Thr Val Thr Lys Ile Arg Glu Thr Tyr Pro
               260                 265                 270
   gaa tta aac att att gct gga aac gtg gca aca gct gaa gcg aca aga       864
   Glu Leu Asn Ile Ile Ala Gly Asn Val Ala Thr Ala Glu Ala Thr Arg
           275                 280                 285
   gcg ctt atc gaa gct gga gca gac gtt gtc aaa gtt gga ata ggg cct       912
   Ala Leu Ile Glu Ala Gly Ala Asp Val Val Lys Val Gly Ile Gly Pro
       290                 295                 300
   ggt tca att tgt act aca cgt gtt gta gcc ggg gtg ggt gtt ccg caa       960
   Gly Ser Ile Cys Thr Thr Arg Val Val Ala Gly Val Gly Val Pro Gln
   305                 310                 315                 320
   att aca gca att tat gat tgt gcg act gaa gca aga aaa cac ggc aaa      1008
   Ile Thr Ala Ile Tyr Asp Cys Ala Thr Glu Ala Arg Lys His Gly Lys
                   325                 330                 335
   aca atc atc gcc gac ggt ggg att aaa ttc tct ggc gat atc act aaa      1056
   Thr Ile Ile Ala Asp Gly Gly Ile Lys Phe Ser Gly Asp Ile Thr Lys
               340                 345                 350
   gca ttg gca gcc ggc gga cat gct gtt atg ctc gga agc ttg ctt gca      1104
   Ala Leu Ala Ala Gly Gly His Ala Val Met Leu Gly Ser Leu Leu Ala
           355                 360                 365
   ggc aca tca gaa agc cct ggt gaa act gaa atc tac caa ggc aga aga      1152
   Gly Thr Ser Glu Ser Pro Gly Glu Thr Glu Ile Tyr Gln Gly Arg Arg
       370                 375                 380
   ttt aag gta tac cgc ggc atg gga tca gtt gct gca atg gaa aaa gga      1200
   Phe Lys Val Tyr Arg Gly Met Gly Ser Val Ala Ala Met Glu Lys Gly
   385                 390                 395                 400
   agt aaa gac cgt tac ttc caa gaa gaa aac aaa aaa ttt gtt cct gaa      1248
   Ser Lys Asp Arg Tyr Phe Gln Glu Glu Asn Lys Lys Phe Val Pro Glu
                   405                 410                 415
   gga att gaa gga cgc aca cct tac aaa ggg cca gtt gaa gaa acc gtt      1296
   Gly Ile Glu Gly Arg Thr Pro Tyr Lys Gly Pro Val Glu Glu Thr Val
               420                 425                 430
   tat cag cta gtc gga ggc ctt cgt tct ggt atg ggg tat tgc ggg tcc      1344
   Tyr Gln Leu Val Gly Gly Leu Arg Ser Gly Met Gly Tyr Cys Gly Ser
           435                 440                 445
   aaa gat ctg cgt gcg cta aga gaa gaa gct cag ttc att cgc atg act      1392
   Lys Asp Leu Arg Ala Leu Arg Glu Glu Ala Gln Phe Ile Arg Met Thr
       450                 455                 460
   ggc gca gga ctt cgc gaa agc cat ccg cat gac gta cag att aca gtg      1440
   Gly Ala Gly Leu Arg Glu Ser His Pro His Asp Val Gln Ile Thr Val
   465                 470                 475                 480
   cat cgt aat aag gcg ctt cct ggt cta ttt ggt tct cat cag aaa aaa      1488
   His Arg Asn Lys Ala Leu Pro Gly Leu Phe Gly Ser His Gln Lys Lys
                   485                 490                 495
   aca gga ttt gtg tat gat gaa tgt tgt caa tcc ggc ttt ttt tca tcg      1536
   Thr Gly Phe Val Tyr Asp Glu Cys Cys Gln Ser Gly Phe Phe Ser Ser
               500                 505                 510
   gat tga                                                              1542
   Asp
<210> 14
   <211> 513
   <212> PRT
   <213> Bacillus subtilis
<400> 14
   Met Trp Glu Ser Lys Phe Ser Lys Glu Gly Leu Thr Phe Asp Asp Val
   1               5                   10                  15
   Leu Leu Val Pro Ala Lys Ser Glu Val Leu Pro His Asp Val Asp Leu
               20                  25                  30
   Ser Val Glu Leu Thr Lys Thr Leu Lys Leu Asn Ile Pro Val Ile Ser
           35                  40                  45
   Ala Gly Met Asp Thr Val Thr Glu Ser Ala Met Ala Ile Ala Met Ala
       50                  55                  60
   Arg Gln Gly Gly Leu Gly Ile Ile His Lys Asn Met Ser Ile Glu Gln
   65                  70                  75                  80
   Gln Ala Glu Gln Val Asp Lys Val Lys Arg Ser Glu Arg Gly Val Ile
                   85                  90                  95
   Thr Asn Pro Phe Phe Leu Thr Pro Asp His Gln Val Phe Asp Ala Glu
   100 105 110
   His Leu Met Gly Lys Tyr Arg Ile Ser Gly Val Pro Ile Val Asn Asn
   115 120 125
   Glu Glu Asp Gln Lys Leu Val Gly Ile Ile Thr Asn Arg Asp Leu Arg
   130 135 140
   Phe Ile Ser Asp Tyr Ser Met Lys Ile Ser Asp Val Met Thr Lys Glu
   145 150 155 160
   Glu Leu Val Thr Ala Ser Val Gly Thr Thr Leu Asp Glu Ala Glu Lys
   165 170 175
   Ile Leu Gln Lys His Lys Ile Glu Lys Leu Pro Leu Val Asp Asp Gln
   180 185 190
   Asn Lys Leu Lys Gly Leu Ile Thr Ile Lys Asp Ile Glu Lys Val Ile
   195 200 205
   Glu Phe Pro Asn Ser Ser Lys Asp Ile His Gly Arg Leu Ile Val Gly
   210 215 220
   Ala Ala Val Gly Val Thr Gly Asp Thr Met Thr Arg Val Lys Lys Leu
   225 230 235 240
   Val Glu Ala Asn Val Asp Val Ile Val Ile Asp Thr Ala His Gly His
   245 250 255
   Ser Gln Gly Val Leu Asn Thr Val Thr Lys Ile Arg Glu Thr Tyr Pro
   260 265 270
   Glu Leu Asn Ile Ile Ala Gly Asn Val Ala Thr Ala Glu Ala Thr Arg
   275 280 285
   Ala Leu Ile Glu Ala Gly Ala Asp Val Val Lys Val Gly Ile Gly Pro
   290 295 300
   Gly Ser Ile Cys Thr Thr Arg Val Val Ala Gly Val Gly Val Pro Gln
   305 310 315 320
   Ile Thr Ala Ile Tyr Asp Cys Ala Thr Glu Ala Arg Lys His Gly Lys
   325 330 335
   Thr Ile Ile Ala Asp Gly Gly Ile Lys Phe Ser Gly Asp Ile Thr Lys
   340 345 350
   Ala Leu Ala Ala Gly Gly His Ala Val Met Leu Gly Ser Leu Leu Ala
   355 360 365
   Gly Thr Ser Glu Ser Pro Gly Glu Thr Glu Ile Tyr Gln Gly Arg Arg
   370 375 380
   Phe Lys Val Tyr Arg Gly Met Gly Ser Val Ala Ala Met Glu Lys Gly
   385 390 395 400
   Ser Lys Asp Arg Tyr Phe Gln Glu Glu Asn Lys Lys Phe Val Pro Glu
   405 410 415
   Gly Ile Glu Gly Arg Thr Pro Tyr Lys Gly Pro Val Glu Glu Thr Val
   420 425 430
   Tyr Gln Leu Val Gly Gly Leu Arg Ser Gly Met Gly Tyr Cys Gly Ser
   435 440 445
   Lys Asp Leu Arg Ala Leu Arg Glu Glu Ala Gln Phe Ile Arg Met Thr
   450 455 460
   Gly Ala Gly Leu Arg Glu Ser His Pro His Asp Val Gln Ile Thr Val
   465 470 475 480
   His Arg Asn Lys Ala Leu Pro Gly Leu Phe Gly Ser His Gln Lys Lys
   485 490 495
   Thr Gly Phe Val Tyr Asp Glu Cys Cys Gln Ser Gly Phe Phe Ser Ser
   500 505 510
   Asp
<210> 15
   <211> 17
   <212> DNA
   <213> Artificial
<220>
   <223> primer
<400> 15
   gaagttgatg atcaaaa 17
<210> 16
   <211> 19
   <212> DNA
   <213> Artificial
<220>
   <223> primer
<400> 16
   acatattgtt gacgataat 19
<210> 17
   <211> 28
   <212> DNA
   <213> Artificial
<220>
   <223> primer
<400> 17
   ttcccttagg gttattttcg tttcaaaa 28
<210> 18
   <211> 50
   <212> DNA
   <213> Artificial
<220>
   <223> primer
<400> 18
   cgtttgttga actaatgggt gcttttatga gcatgtgcat gataaggtga 50
<210> 19
   <211> 50
   <212> DNA
   <213> Artificial
<220>
   <223> primer
<400> 19
   acagctccag atccatatcc ttctttttta gagagtttgc gggagtatcg 50
<210> 20
   <211> 27
   <212> DNA
   <213> Artificial
<220>
   <223> primer
<400> 20
   taaaggtttt tcgggataag attgaaa 27
<210> 21
   <211> 50
   <212> DNA
   <213> Artificial
<220>
   <223> primer
<400> 21
   tcaccttatc atgcacatgc tcataaaagc acccattagt tcaacaaacg 50
<210> 22
   <211> 50
   <212> DNA
   <213> Artificial
<220>
   <223> primer
<400> 22
   cgatactccc gcaaactctc taaaaaagaa ggatatggat ctggagctgt 50